# EUROPEAN PATENT APPLICATION

(11) **EP 2 982 368 A1**
(43) Date of publication of application: **10.02.2016**
(21) Application number: 14179698.7
(22) Date of filing: 04.08.2014
(51) Int. Cl.: A61K 31/04, A61K 31/05, A61K 31/085, A61K 31/135, A61K 31/216, A61K 31/341, A61K 31/381, A61K 31/404, A61K 31/4406, A61K 31/655, A61P 35/00, A61P 9/00

(54) **Small molecule compounds displacing focal adhesion core components**

(71) Applicant: Universität Konstanz, 78464 Konstanz (DE)
(72) Inventor: Buntru, Alexander, 13187 Berlin (DE); Hauck, Christof, 78465 Konstanz (DE); Mayer, Thomas, 78464 Konstanz (DE); Paone, Christoph, 78467 Konstanz (DE); Strittmatter, Tobias, 78467 Konstanz (DE)
(74) Representative: PATERIS Patentanwälte PartmbB

(57) **Abstract**

The present invention relates to compounds of Formula I for use in inhibiting cell migration. Further, the invention relates to a compound of Formula I for use in preventing and/or treating restenosis, for use in treating cancer and for use in preventing and/or treating cardiovascular diseases. Likewise, the invention relates to the use of a compound of Formula I for inhibiting cellular migration and/or the turnover of focal adhesions *in vitro.*

## Description

### Field of the invention

The present invention relates to compounds of Formula I for use in inhibiting cell migration. Further, the invention relates to a compound of Formula I for use in preventing and/or treating restenosis, for use in treating cancer and for use in preventing and/or treating cardiovascular diseases. Likewise, the invention relates to the use of a compound of Formula I for inhibiting cellular migration and/or the turnover of focal adhesions *in vitro.*

### Background of the invention

The regulation of cell proliferation, survival and motility is often found to be critical for the change from physical to pathological conditions of a cell, a tissue or an entire organ. Under physiological conditions, for example, cell adhesion and migration are highly regulated processes, which play roles in tissue formation, embryonic development, wound healing as well as immune responses. Accordingly, defects in the process of cellular adhesion and/or migration are found in various diseases and pathological conditions, such as cancer, in particular tumor metastasis and vascularization, in cardiovascular malfunctions and immunological disorders. Although diverse cellular mechanisms contribute to these diseases and disorders, integrin-based focal adhesions constitute the central cellular structures, which orchestrate cell adhesion and/or cell migration. This is likely to be because focal adhesions (FAs) provide the structural link between the extracellular matrix and the intracellular actin cytoskeleton in the form of actin stress fibers. Furthermore, FAs also serve as key signaling hubs for adhesion dependent proliferation and survival of adherent cell types, as well as for regulation of cell motility. FAs are multicomponent protein complexes, which are initiated upon integrin-mediated contact with extracellular matrix proteins (Schiller HB et al., 2011). Several characteristic cellular proteins, such as talin, paxillin or α-actinin, directly associate with integrin cytoplasmic domains and trigger the hierarchical recruitment and incorporation of additional components into the maturing focal adhesion plaque (Kanchanawong P et al., 2010). One important talin- and paxillin-binding protein is the focal adhesion kinase (FAK), a FA-localized non-receptor tyrosine kinase. This enzyme is key for post-translational modification of integrin-associated proteins and the dynamic regulation of FA composition and functionality (Hauck et al., 2002a). Together with its binding partners talin and paxillin, FAK is one of the core constituents of focal adhesions and instrumental for their regulated assembly and turnover. For example, fibroblasts derived from FAK-deficient mouse embryos (FAK -/-) have an increased number of focal adhesion and actin stress fibers and display impaired focal adhesion turnover kinetics. Furthermore, these cells exhibit a dramatically reduced potential of haptotactic, chemotactic and mechanotactic migration. Similarly, paxillin-deficient fibroblasts show defects in focal adhesion site formation and turnover (Hagel M et al., 2002; Wade R et al., 2002). It is interesting to note, that FAK and paxillin depend on each other, as FAK is recruited to integrin-rich focal adhesion sites via paxillin and paxillin in turn serves as a substrate for FAK (Deramaudt TB et al., 2014). Therefore, interference with FAK or paxillin could be a strategy to modulate cell adhesion and/or migration in pathological settings.

As FAK possesses enzyme activity and can autophosphorylate a critical tyrosine residue (Y397) located between the amino-terminal FERM and the kinase domains, pharmacological FAK inhibitors have been developed (Liu TJ et al., 2007; Golubovskaya VM et al., 2008; Golubovskaya VM, 2012). However, FAK primarily functions as an assembly platform for a focal adhesion-based signaling complex regulating adhesion, migration and other cellular processes. Most importantly, FAK kinase activity is not essential for this assembly function as FAK can be effectively transphosphorylated by other cellular tyrosine kinases such as activated receptor tyrosine kinases (Sieg DJ et al., 2000). Therefore, additional means to interfere with the scaffolding function of FAK are required. A physiological way to modulate FAK function is via expression of a separate transcript from the FAK gene, the so-called FAK-related non-kinase (FRNK), which has been observed in some cell types (Taylor JM et al., 2001). The FRNK transcript is initiated from an internal promoter in the FAK gene and comprises the carboxy-terminal domain of FAK harbouring the paxillin- and talin-binding sites. Expression of FRNK displaces FAK from its subcellular binding sites at focal adhesions thereby effectively inhibiting cellular motility without compromising cell viability. Previous studies have also demonstrated that expression of FRNK in adenocarcinoma cells and transformed fibroblasts can strongly reduce cancer cell invasion in vitro and in vivo (Hauck CR et al., 2002b; Hauck CR et al., 2001). These investigations suggest that displacing FAK from focal adhesion sites is an efficient means to selectively interfere with the motility-promoting functions of FAK. However, FRNK is a protein of about 360 amino acids and, thus, cannot be introduced easily into a living cell from outside. Instead, FRNK needs to be expressed within the target cell, which can only be achieved by gene therapeutic approaches (EP 1 644 509).

Therefore, there is a need for compounds, which interfere with FAK localization at focal adhesions and which can be directly and easily administered to target cells.

### Summary of the invention

In a first aspect, the present invention relates to a compound of Formula I 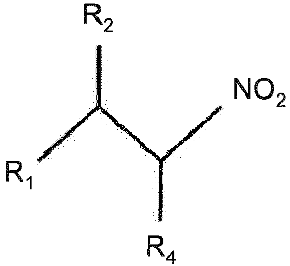 for use in inhibiting cell migration, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

In a further aspect, the present invention relates to a compound of Formula I 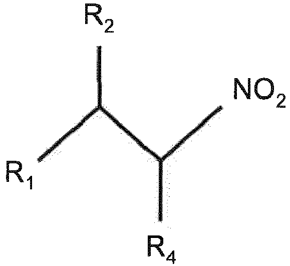 for use in preventing and/or treating restenosis, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

In a further aspect, the invention relates to a compound of Formula I 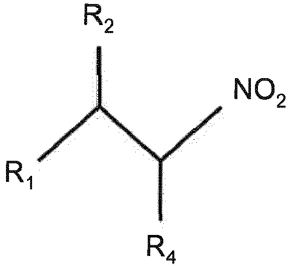 for use in treating cancer, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

In a further aspect, the present invention relates to a compound of Formula I 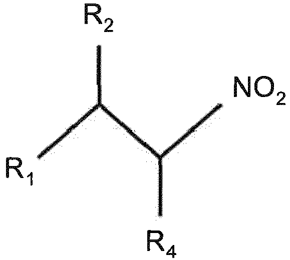 for use in preventing and/or treating cardiovascular diseases, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

Further, a compound of Formula I 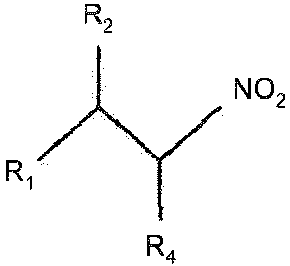 for use in treating inflammation, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing, is disclosed.

In a further aspect, the invention relates to the use of a compound of Formula I 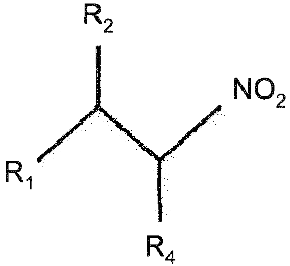 for inhibiting cellular migration and/or the turnover of focal adhesions *in vitro,* wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

### Brief description of the drawings

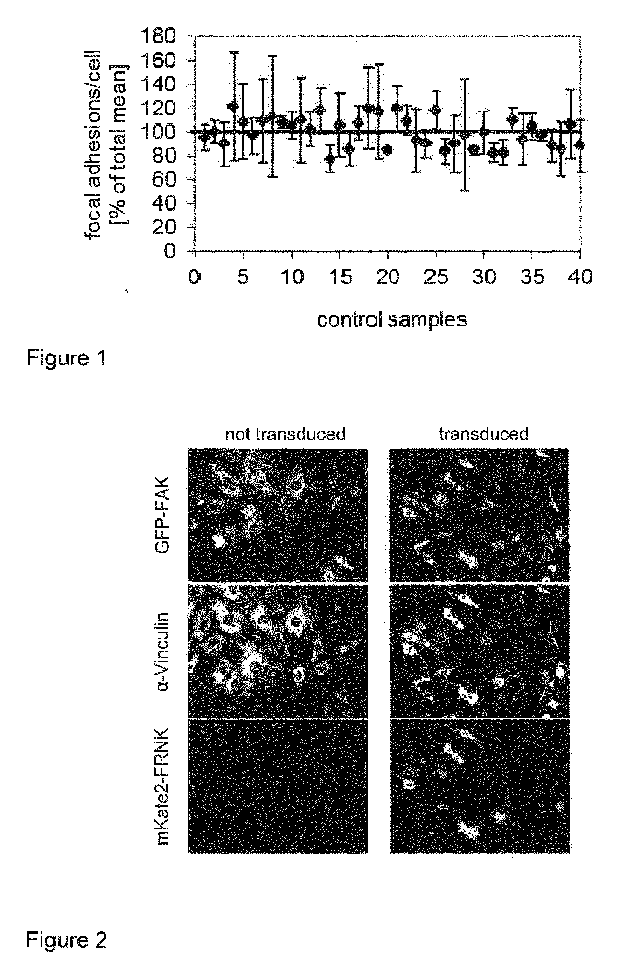

Figure 1 shows software-based quantification of focal adhesions. Image J was used to automatically quantify FAK-containing focal adhesions due to local maxima of fluorescence intensity in the GFP-channel (5 to 70 pixel). The number of cells was automatically counted in the Hoechst channel. To test reproducibility of the system 40 control wells were quantified wherein four positions for each well were imaged. The mean of all found adhesions per cell were set to 100 %.
Figure 2 shows GFP-FAK mouse embryonic fibroblasts, which were seeded on fibronectin and transduced to express mKate2-tagged FRNK, vinculin was visualized by antibody staining to have a FAK independent marker of focal adhesions.

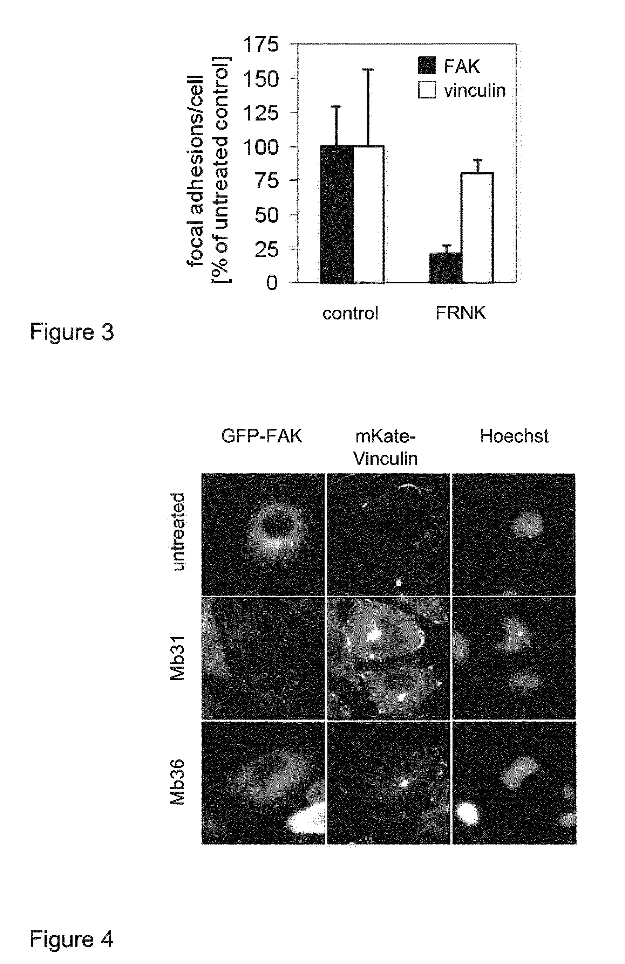

Figure 3 shows the quantification of focal adhesions in FRNK transduced cells vs control. Focal adhesions per cell were quantified using GFP-FAK and anti-vinculin antibody staining in FRNK-transduced and untreated cells.
Figure 4 shows the displacement of FAK from focal adhesions by Mb31 and Mb36, while vinculin localization is not disturbed. GFP-FAK mouse embryonic fibroblasts stably expressing mKate-vinculin were incubated with 40 µM of each small molecule compound, respectively.

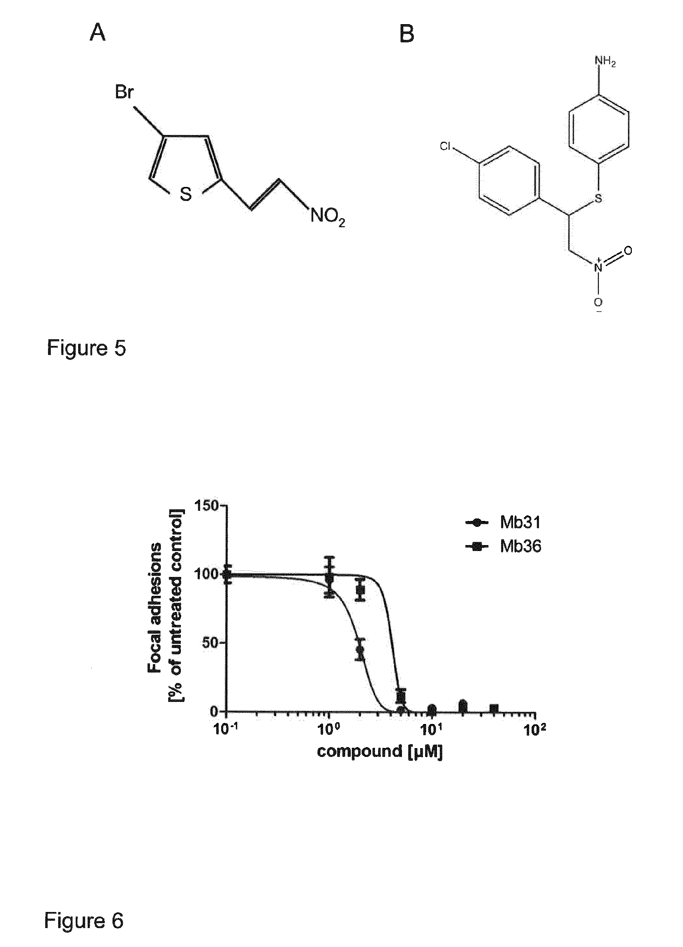

Figure 5 shows chemical formulae for trans-3-4-Bromo-2-(2-nitrovinyl)thiophene (Mb31) (A) and 4-((1-(4-Chlorophenyl)-2-nitroethyl)thio)aniline (Mb36) (B).
Figure 6 shows the displacement of FAK from focal adhesions at low micromolar concentrations of Mb31 and Mb36. GFP-FAK mouse embryonic fibroblasts were seeded on fibronectin and incubated for 1 h with the indicated concentrations of the small compounds, respectively. Focal adhesions per cell were quantified to determine the cellular IC₅₀ for each compound.

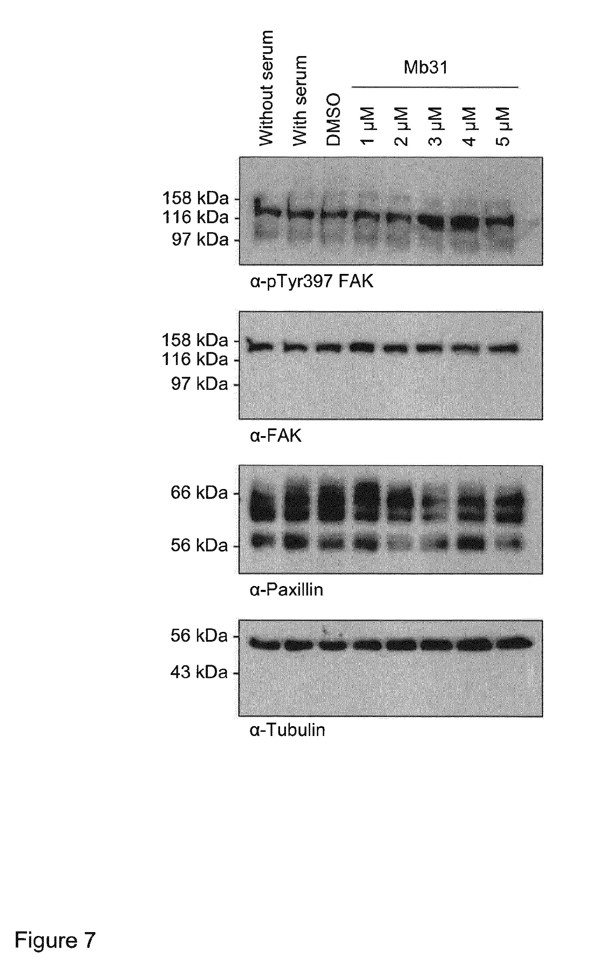

Figure 7 shows that Mb31 does not target FAK kinase activity. GFP-FAK mouse embryonic fibroblasts were seeded on fibronectin and serum-starved overnight. The indicated concentrations of Mb31 were applied for 30 min. Cells were lysed and analyzed for paxillin and FAK protein level as well as FAK Tyr397 phosphorylation. Tubulin served as a loading control.

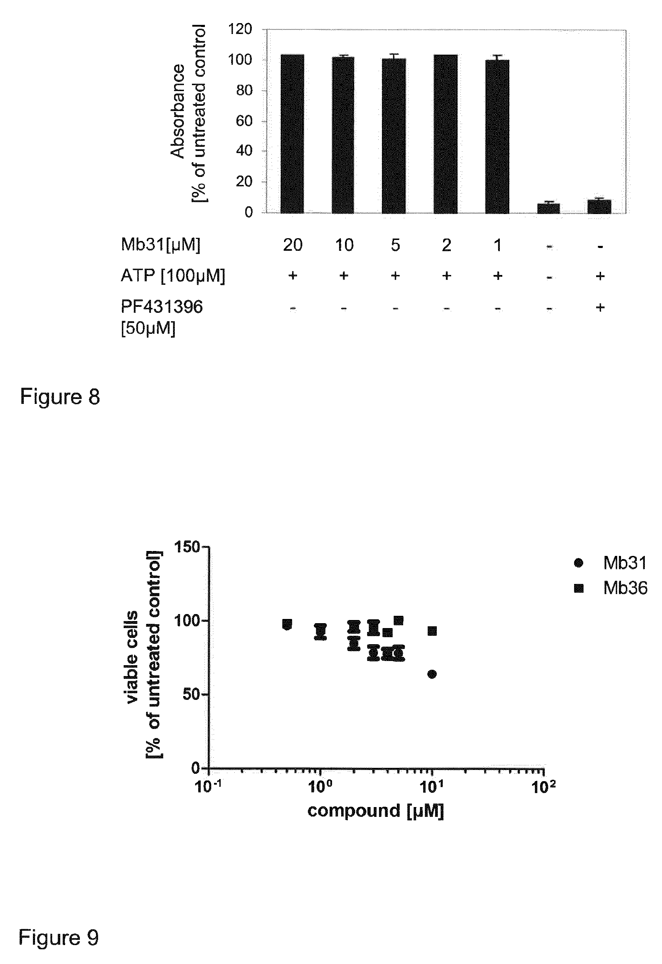

Figure 8 shows the quantification of FAK phosphorylation. The purified kinase domain of FAK was incubated with increasing amounts of Mb31 for 1 h at 30°C together with poly-Glu-Tyr as a FAK substrate. Phosphorylation was quantified by enzyme-coupled color reaction.
Figure 9 shows that Mb31 and Mb36 do not impair cell viability. Serum-starved GFP-FAK mouse embryonic fibroblasts were stimulated with normal growth medium and incubated for 5 h with the indicated concentrations of the small molecule compounds, respectively. Cell viability was assessed by MTT assay. Values represent means of triplicates ± SEM of two independent experiments.

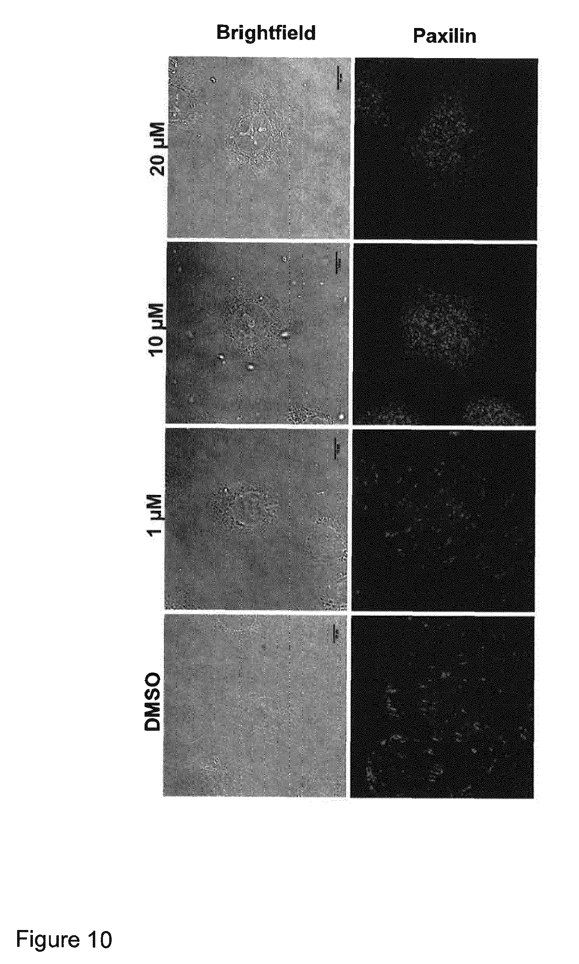

Figure 10 shows the displacement of paxillin in FAK-deficient mouse embryonic fibroblasts (MEF) upon treatment for 30 min with the indicated amounts of Mb31. In solvent (DMSO)-treated cells, paxillin is localized at focal adhesion sites.

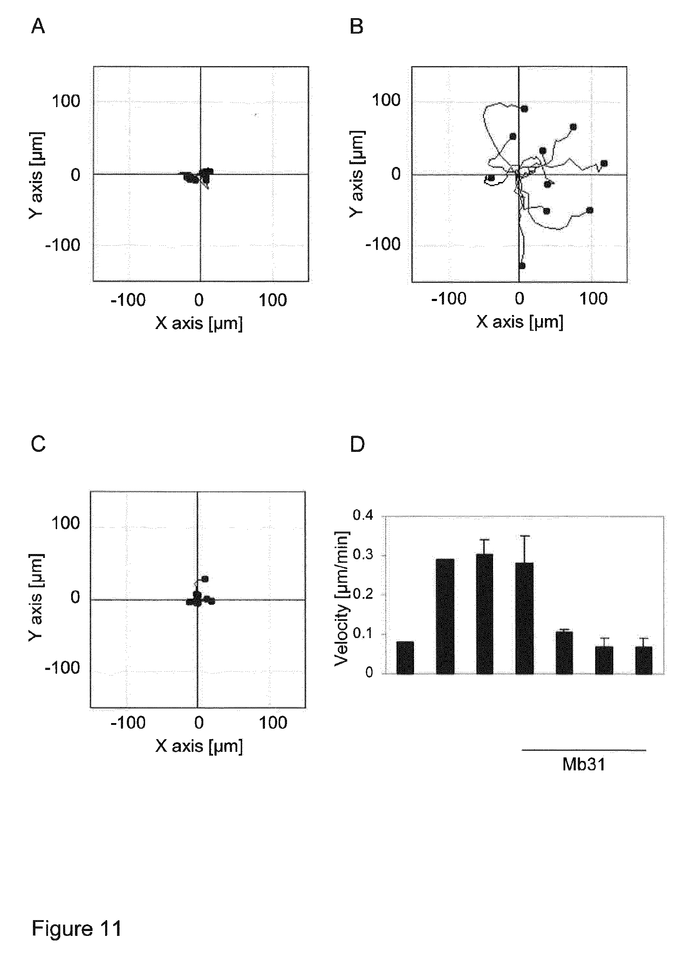

Figure 11 shows the inhibition of integrin based cell migration of fibroblasts by Mb31. GFP-FAK MEFs were seeded on fibronectin and serum-starved overnight. Migration was stimulated by replacing the medium with normal growth medium. Migration of single cells was tracked for 8 h in 30 min intervals. Migration was recorded from cells that were cultured without serum (A), with serum (B) or with serum in the presence of 3 µM Mb31 (C). The velocity of the recorded cells was quantified as shown in (D).

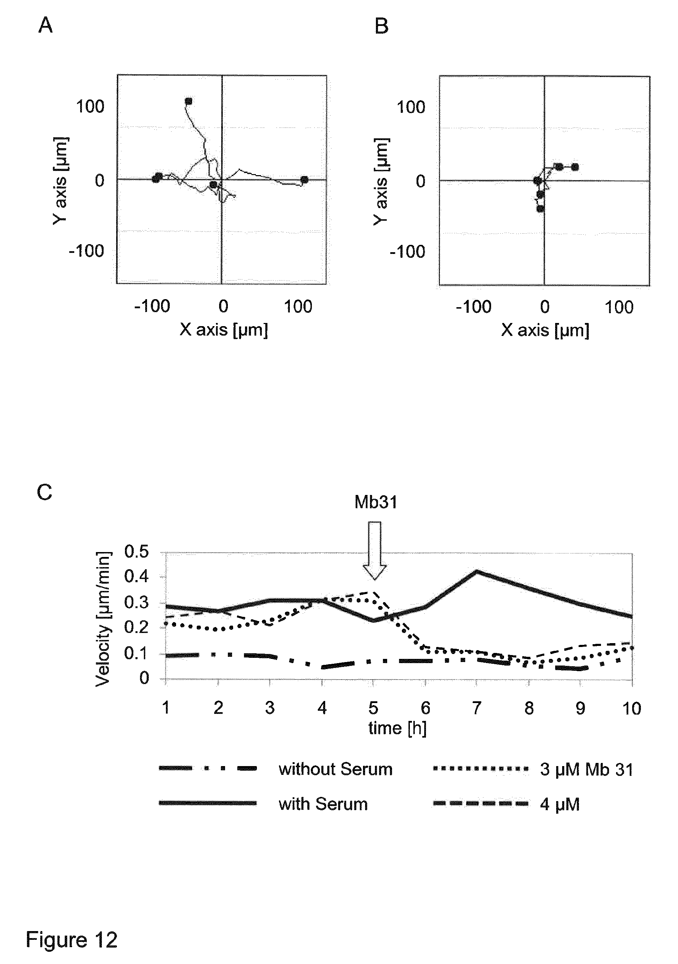

Figure 12 shows inhibition of cell migration upon addition of Mb31. (A) GFP-FAK MEFs were seeded and serum-starved as in Figure 10. Cell migration was stimulated with normal growth medium and single cells were tracked for 5 h. (B) Mb31 was added and the same cells were tracked for additional 5 h. (C) Mean velocities of differently treated cells were plotted over time. After 5 h, Mb31 was applied at the indicated concentrations.

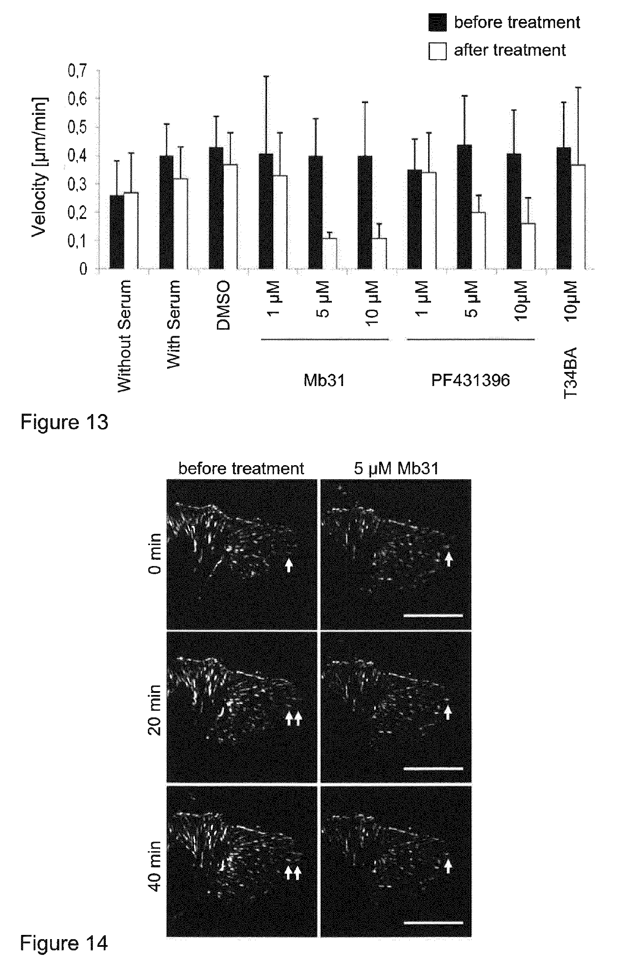

Figure 13 shows that Mb31 restricts EGF-stimulated cell migration of transformed cells. NIH-v-Src fibroblasts were seeded on 2 µg/ml FN in DMEM/0.5%BSA and stimulated with 10 ng/ml EGF or kept unstimulated. 10 cells per sample were tracked for 6 h before (black bars) and 6 h after (white bars) the addition of Mb31, FAK/Pyk2 inhibitor PF431396, or T34BA (trans-3-(4-Bromothiophen-2-yl)acrylic acid. Mb31 and PF431396 are able to efficiently inhibit the migration of EGF-stimulated cells, whereas T34BA shows no effect.
Figure 14 shows the Effect of Mb31 on paxillin displacement is independent of FAK. FAK-/- MEF cells were treated for 30 min with 1, 10, or 20 µM Mb31, fixed, immuno-stained against paxillin and analyzed via confocal microscopy. DMSO treatment served as a vehicle control. In DMSO treated cells, paxillin is clearly detectable at focal adhesion sites, but is efficiently displaced by Mb31 concentrations >1 µM.

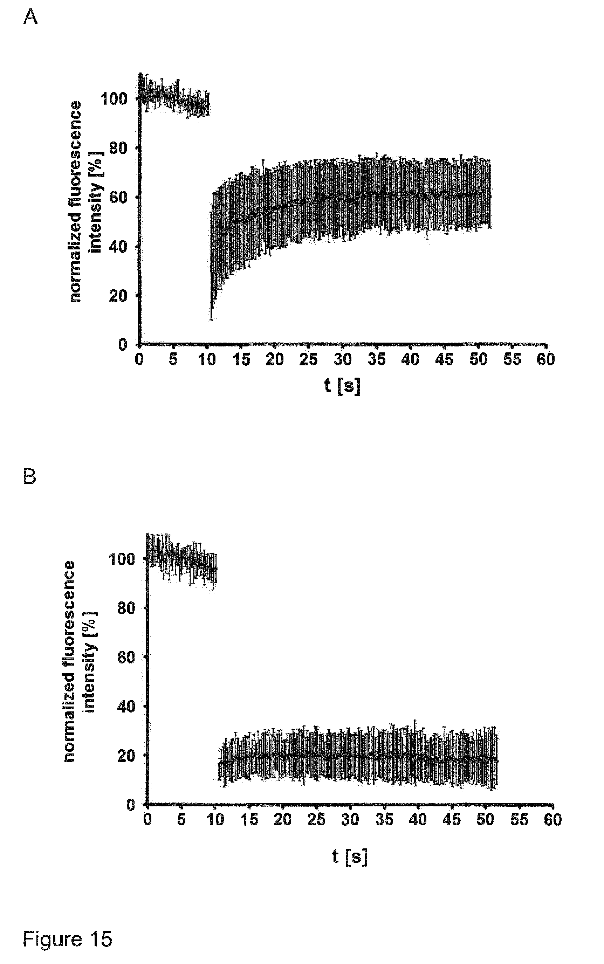

Figure 15 shows protein dynamics in focal adhesions upon treatment with Mb31. Mouse embryonic fibroblasts (MEFs) were transfected with talin-mCherry and talin dynamics analyzed in focal adhesions using fluorescence recovery after photobleaching (FRAP). Cells were left untreated (A) or treated with 10 µM of Mb31 (B). In untreated cells, the mCherry intensity recovered, indicating replacement of bleached by non-bleached talin and therefore turnover of talin at focal adhesions. Treatment of cells with the small molecule compound Mb31 resulted in a lack of fluorescent recovery indicating a lack of talin turnover.

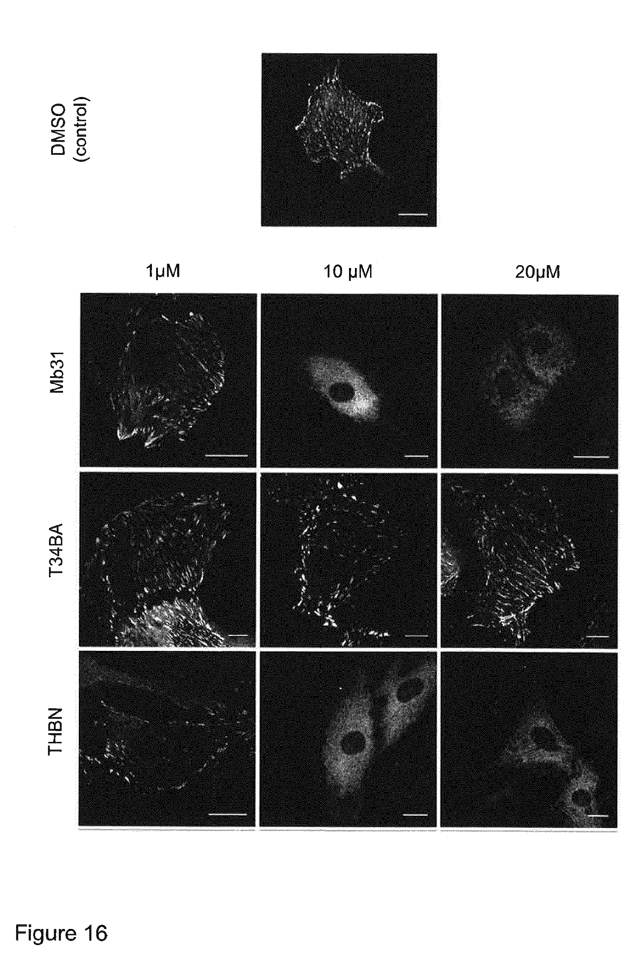

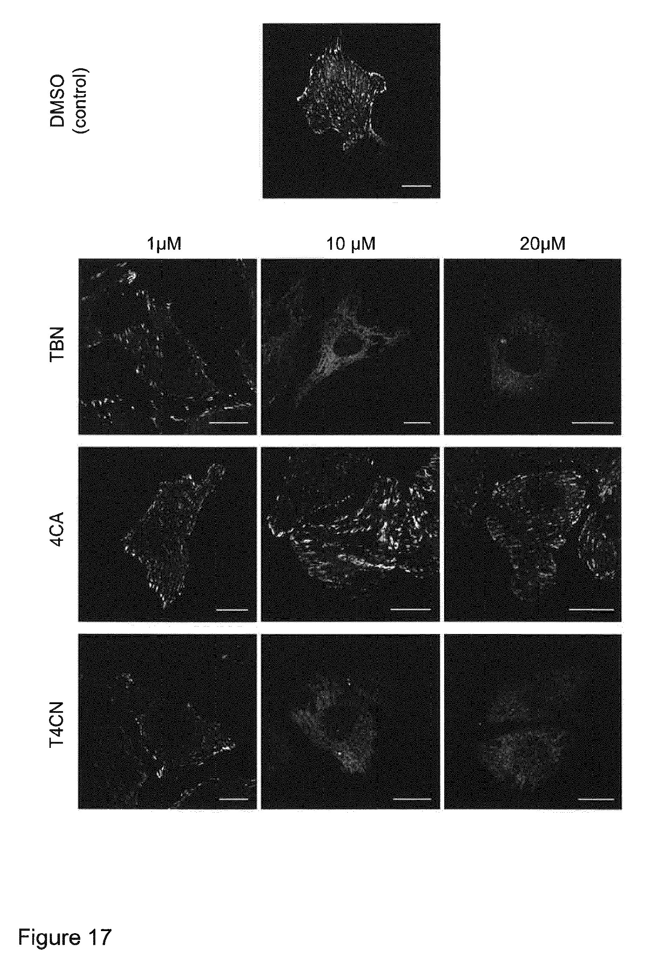

Figure 16 and 17 show the displacement of FAK from focal adhesions by different small molecule compounds of related structure: trans-3-4-Bromo-2-(2-nitrovinyl)thiophene (Mb31, also referred to as FLocker1), (*trans*-3-Hydroxy-β-nitrostyrene) (THBN, also referred to as FLocker3), (*trans*-β-Nitrostyrene) (TBN), (*trans*-4-Chloro-β-nitrostyrene) (T4CN), *trans*-3-(4-Bromothiophen-2-yl)acrylic acid (T34BA) and 4-Chlorocinnamic acid (4CA). Mouse fibroblasts stably expressing GFP-FAK were treated for 30 min with 1, 10 or 20 µM of the indicated compounds and analyzed for GFP-FAK localization.

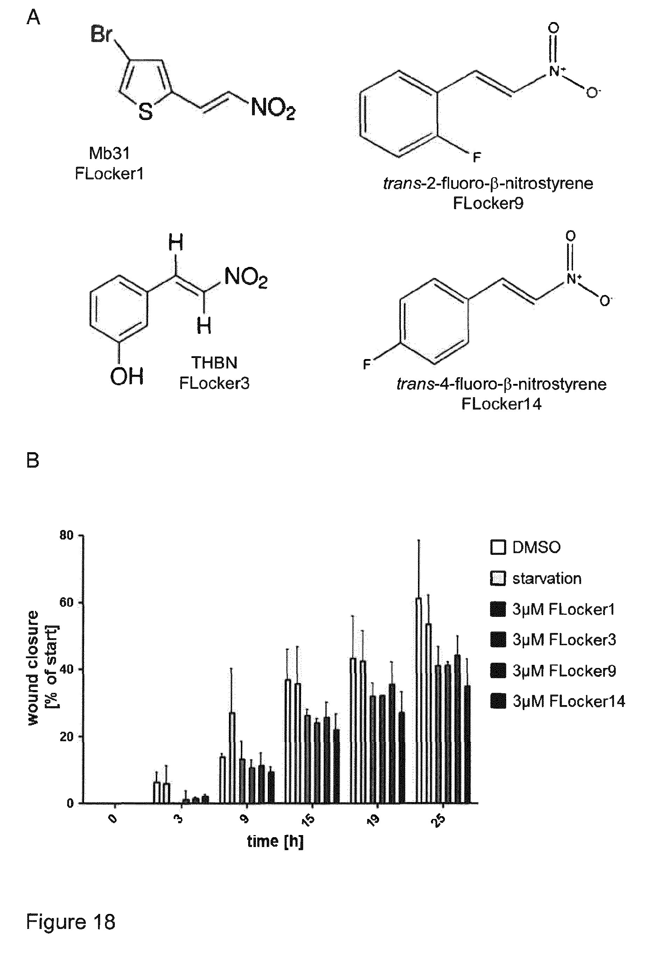

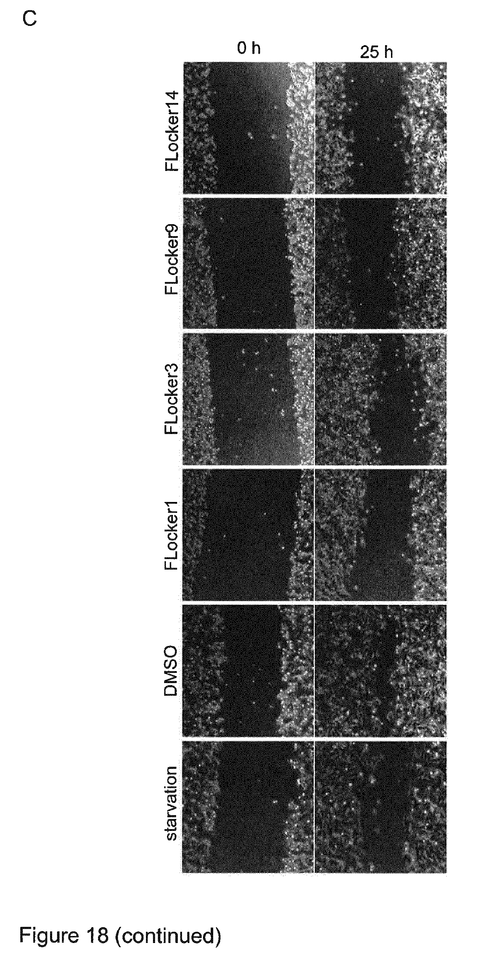

Figure 18 shows the inhibition of cancer cell migration by Mb31. (A) Structures of Mb31 (also referred to as FLocker1, for its ability to *"lock the feet of the cell"*) and additional FLocker compounds. (B) Confluent monolayers of MDA-MB-231 breast cancer cells were serum starved (0.5% BSA in DMEM) for 15 h. A wound was scratched using a sterile yellow pipet tip. Detached cells were removed by washing with PBS and the wounded monolayer was photographed (0 h). The cells were further incubated in either starvation medium, growth medium containing vehicle control (DMSO) or growth medium containing 3 µM Foot Locker (FLocker 1, 3, 9 or 14) compounds. After this incubation, the same areas of the wound were again photographed (25 h). (C) Wound closure was microscopically analyzed and evaluated using ImageJ software.

### Detailed description of the invention

In a first aspect, the present invention relates to a compound of Formula I 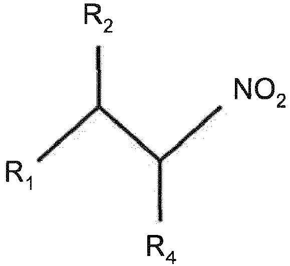 for use in inhibiting cell migration, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

The small molecule compounds of the invention effectively disrupt protein-protein interactions at focal adhesion sites leading to a delocalization of FAK and paxillin from focal adhesions. As visualized by the expression of a FAK-GFP fusion protein in FAK deficient mouse embryo fibroblasts or by paxillin immunostaining, administration of the compounds of the invention results in a relocalization of FAK and paxillin from the focal adhesions to the cytoplasm (e.g. **Figures 10****,** **16****, and** **17****).** FAK is a ubiquitously expressed 125 kDa non-receptor protein tyrosine kinase (PTK) and is highly conserved across species. Structurally, FAK comprises three domains and localizes at sites of clustered integrins, so called focal adhesions. FAK and paxillin are two of the first proteins recruited to sites of nascent adhesion assembly, where paxillin seems to first recruit FAK and than FAK mediates the recruitment of talin to stabilize the growing adhesions. Together with inducing FAK and paxillin delocalization, the compounds of the invention exert a significant effect on cell migration. Upon treatment with small molecules of the invention, cells become largely immobile **(****Figure 11****).** Closer investigations revealed that upon administration of a compound of the invention cells stop to form nascent adhesions while at the same time pre-existing mature adhesions become immobile. This indicates that the compounds interfere with both, assembly and disassembly of focal adhesions. These effects were observed upon administration of compounds of the invention in concentrations as low as 2 µM.

Interestingly, although promoting delocalization of FAK and paxillin from existing focal adhesions and blocking focal adhesion turnover, focal adhesions per se are maintained. This was evident as e.g. vinculin and talin remained localized at the points of focal adhesion (see also Table 1). Thus, while inhibiting cell motility, the compounds of the invention do not impair the cells anchorage within their tissue. Likewise, the cells' physiological morphology and their cytoskeletal organization are maintained. This is consistent with the observation that the small molecule compounds of the invention do not induce degradation of FAK or paxillin, since both molecules re-localize to the focal adhesions upon removal of the compound. Likewise, the small molecule compounds of the invention do not interfere with the kinase function of FAK, such that other functions of FAK, as e.g. regulating gene expression and chromatin remodeling in the nucleus (Schaller, 2010), should remain unaffected. Accordingly, it was found that the compounds of the invention do not exhibit toxic effects on the cells in those concentrations and time scales affecting cell migration. This is of particular importance, since it demonstrates the specificity of the compounds. Thus, the compounds of the invention provide efficient instruments to target FAK and paxillin, which additionally exhibit a low probability of causing side effects. Moreover, they provide precise tools for *in vitro* research.

In summary, the compounds of the invention specifically displace FAK and paxillin from focal adhesion, already at particularly low concentrations and in a reversible manner while localization of other focal adhesion proteins is not disturbed. As a result, they efficiently block both nascent adhesion establishment as well as focal adhesion turnover leading to an inhibition of integrin based cell migration.

By investigating various small molecule compounds of related structure it was revealed that compounds showing an efficient and specific effect on FAK localization, comprise an aromatic residue (R₁) linked by a chain of two carbon atoms to a nitro functional group. Further residues and substitutions, however, although potentially influencing the compounds' effective concentration and time to effect, may vary. The term "aromatic group" as used herein refers to a chemical group derived from a structure having a cyclic, delocalized (4n+2) pi-electron system (theory of Hückel; IUPAC), comprising e.g. arenes and their substitution product such as benzene, naphthalene, toluene and cyclopentadien, and aromatic heterocyclic structures, such as thiophenene and pyridine.

In a preferred embodiment R₁ and/or R₃ are independently an aryl or heteroaryl. The term "aryl" as used herein refers to a group derived from an arene by removal of a hydrogen atom from a ring carbon. Aryls comprise e.g. phenyl, naphthyl, benzyl. The term "heteroaryl" as used herein refers to a group derived from a heteroarene (i.e. a compound derived from an arene by replacement of one or more methine and/or vinylene groups by divalent or trivalent heteroatoms, respectively, in such way as to maintain an aromatic system) by removal of a hydrogen atom from any ring atom. Heteroaryls comprise e.g. pyridyl, indoyl, thiophenyl, pyrrolyl.

In a further preferred embodiment, the aryl or heteroaryl is monocyclic, bicyclic or tricyclic.

In a preferred embodiment, the aryl or heteroaryl comprises 4 to 18 ring members, preferably 5, 6, 9 or 10 ring members.

In a preferred embodiment, the heteroaryl comprises 1 to 5 heteroatoms, preferably 1 or 2 heteroatoms, further preferred selected from the group consisting of S, N and O.

In a preferred embodiment, the aryl or heteroaryl is unsubstituted.

In an alternative embodiment, the aryl or heteroaryl is substituted and the substituent is selected from the group consisting of hydroxyl, alkyl, preferably methyl, ethyl or butyl, aryl, preferably monocyclic, bicyclic or tricyclic aryl, heteroaryl, sulfanyl and halogen, preferably Br, Cl or F.

In a preferred embodiment, R₁ is a monocyclic, optionally substituted aryl or heteroaryl and R₂ and R₄ are missing. Such compounds were found to induce a particularly fast displacement of FAK from focal adhesions. FAK displacement by such small molecule compounds was observed within 5 min at concentrations of 20 µM. Likewise, such compounds induced FAK delocalization at particularly low concentrations, e.g. as low as 2 µM.

In a preferred embodiment, the compound is selected from the group consisting of 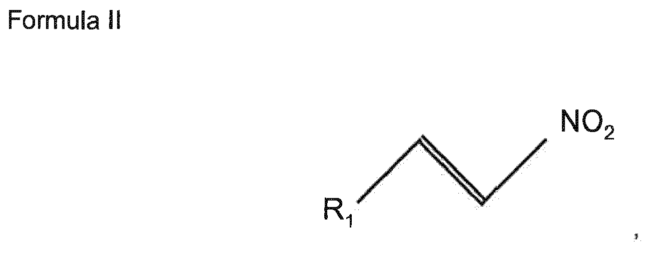 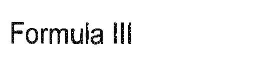 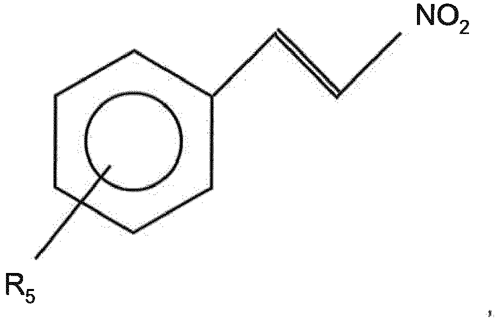 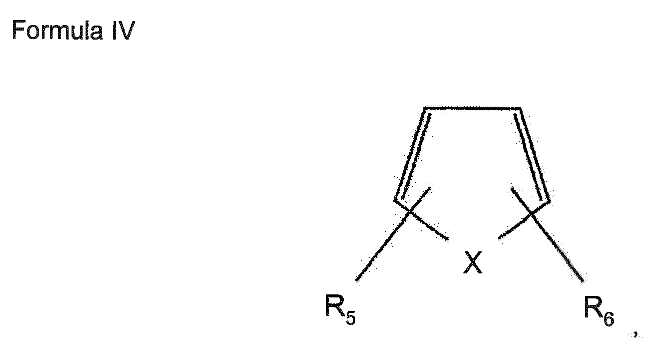 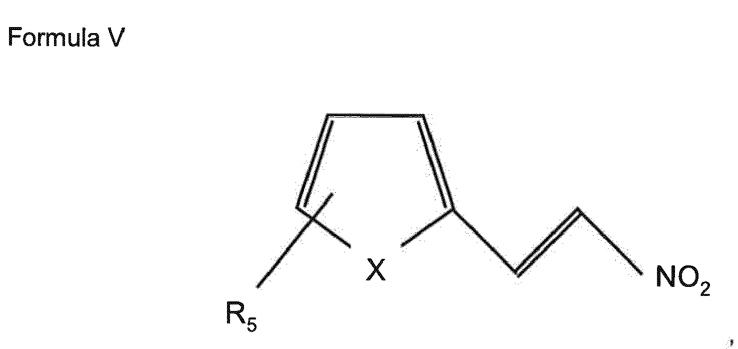 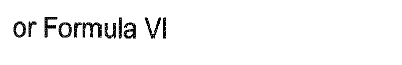 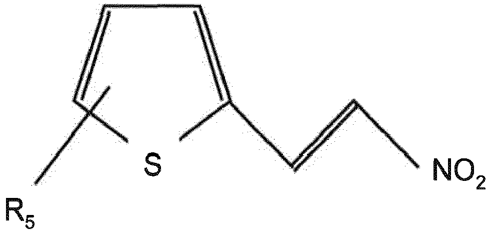 wherein R₁ is an aromatic group, X is S, O or N, R₅ is hydroxyl, alkyl, preferably methyl, ethyl or butyl, aryl, preferably monocyclic, bicyclic or tricyclic aryl, heteroaryl, sulfanyl and halogen, preferably Br or Cl, or missing and R₆, if present, is

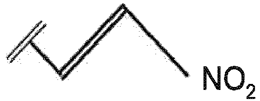

In an alternative embodiment, R₁ is a monocyclic, optionally substituted aryl or heteroaryl and R₄ is hydrogen.

In a further preferred embodiment, the compound is selected from the group consisting of Formula VII, 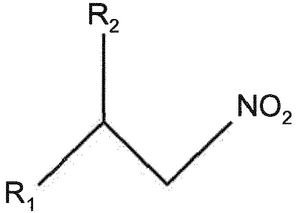 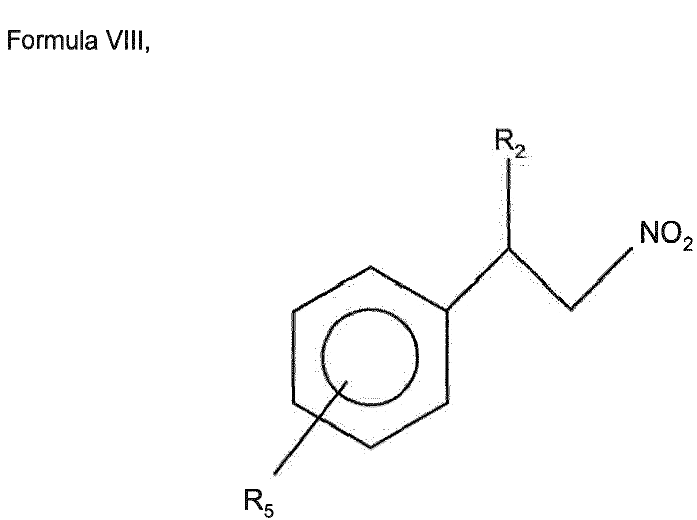 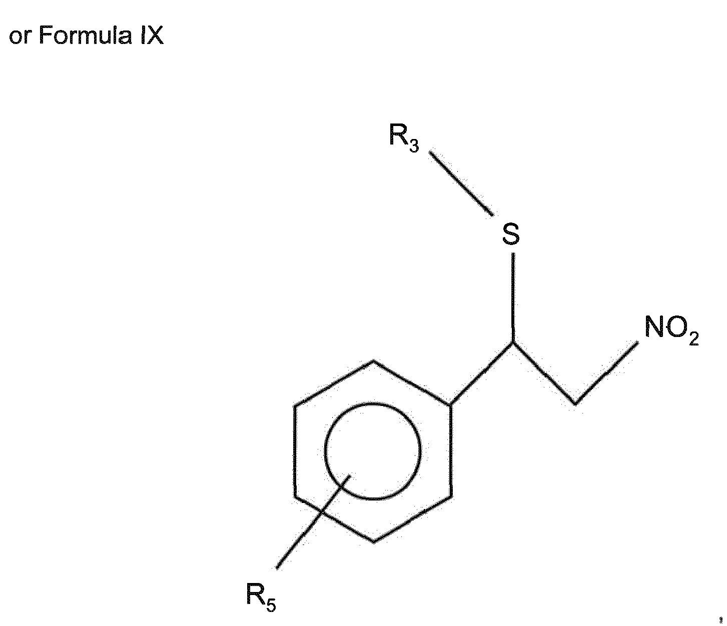 wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₅ is hydroxyl, alkyl, preferably methyl, ethyl or butyl, aryl, preferably monocyclic, bicyclic or tricyclic aryl, heteroaryl, sulfanyl and halogen, preferably Br or Cl, or missing.

In a preferred embodiment, the IC₅₀ of the compound is about 0,1 - about 0,5 µM, preferably about 0,1 - about 0,4 µM, more preferred about 0,1 - about 0,25 µM. Compounds having a low IC₅₀ and showing fast kinetics are particularly preferred since they are already effective at low concentrations and cause a fast FAK delocalization. The IC₅₀ values of Mb31 and Mb36, are about 2.0 µM and about 4.1 µM, respectively.

In a preferred embodiment, the compound has a molecular mass of 1.500 Da or less, preferably 1.000 Da or less, more preferred 900 Da or less. Small molecule compounds are usually defined as chemical compounds having a molecular mass of less than about 1.000 or 900 Da. For entering a cell and exerting its biological activity, however, a compound might be as large as 1.500 Da.

In a preferred embodiment, the compound is selected from the group consisting of 4-bromo-2-(2-nitrovinyl) thiophene (Mb31), 4-{[1-(4-chlorophenyl)-2-nitroethyl]thio}aniline (Mb36), (E)-3-(2-nitrovinyl)pieno, (E)-1-methoxy-3-(2-nitrovinyl)benzene, (E)-ethyl 6-(4-(2-nitrovinyl)phenoxy)hexanoate, (E)-N-(2-(2-(2-(2-azidoethoxy)ethoxy)ethoxy)ethyl)-7-(3-(2-nitrovinyl)phenoxy)heptanamide, (E)-2-(2-nitrovinyl)pieno, (E)-1-fluoro-2-(2-nitrovinyl) benzene, (E)-1-methyl-4-(2-nitrovinyl) benzene, (E)-1-bromo-3-(2-nitrovinyl)benzene, (E)-1-chloro-3-(2-nitrovinyl)benzene, (E)-1-nitro-3-(2-nitrovinyl)benzene, (E)-1-fluoro-4-(2-nitrovinyl)benzene, (E)-(2-nitroprop-1-en 1-yl)benzene, 1-bromo-4-[(1E)-2-nitro-1-propenyl]benzene, 2-(2-Nitrovinyl)-furan, and 3-(2-nitro-vinyl)-pyridine. All of these compounds were found to efficiently disrupt protein-protein interactions at focal adhesion site with the following compounds showing the most prominent effect: 4-bromo-2-(2-nitrovinyl) thiophene (Mb31), 4-{[1-(4-chlorophenyl)-2-nitroethyl]thio}aniline (Mb36), (E)-3-(2-nitrovinyl)pieno, (E)-1-methoxy-3-(2-nitrovinyl)benzene, (E)-1-fluoro-2-(2-nitrovinyl) benzene, (E)-1-methyl-4-(2-nitrovinyl) benzene, (E)-1-nitro-3-(2-nitrovinyl)benzene, (E)-1-fluoro-4-(2-nitrovinyl)benzene, 1-bromo-4-[(1E)-2-nitro-1-propenyl]benzene, and 2-(2-Nitrovinyl)-furan.

In a preferred embodiment, the cell is a eukaryotic cell, preferably a mammalian cell, more preferred a human cell. The compounds of the invention target FAK and paxillin at focal adhesions. Both molecules are highly conserved between different species. Therefore, the compounds are suitable for inhibiting cell migration in cells of different species, in particular of different mammalian species including human.

In a preferred embodiment, the cell is a vascular muscle cell, a cell of the immune system and/or a cancer cell. Cell motility varies between different cell types with some cells being particularly agile as e.g. vascular muscle cells and cells of the immune system. Those cells move within tissues or even throughout the entire body having the particular capability of invading tissue. Other cell types, as e.g. cancer cells, become motile only in the course of pathological modifications leading to the migration and invasion of the cells through and into tissues. This transformation was found to be crucial for the development of metastases. Using the compounds of the invention, cell migration can be inhibited, which is of particular interest in the case of pathological migration behavior.

In a further aspect, the invention relates to a compound of Formula I 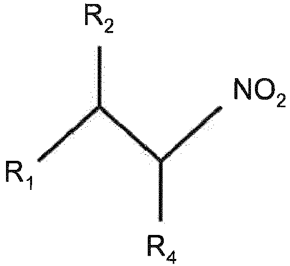 for use in preventing and/or treating restenosis, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

Restenosis often occurs in the course of wound healing after implantation of a stent leading to the thickening of the vessel wall and finally to the occlusion of the entire vessel, which should have actually been opened by the stent. It involves diverse cellular processes including inflammation, extracellular matrix remodeling, muscle cell proliferation and cell migration. Extracellular matrix remodeling as well as cell migration are both dependent on focal adhesions' assembly and disassembly. These processes can be interfered with, in particular inhibited, by the compounds of the invention. Thus, the compounds are especially suitable for treating restenosis.

For example, when restenosis is initiated upon stenting, an inflammatory process is started in which leukocytes adhere to and "roll" along the insured vessel's surface before they migrate through the endothelial cell layer. This initial process of restenosis is highly dependent on the formation of adhesions between leukocytes and endothelial cells. Inhibiting the formation of nascent focal adhesions and the migration of leukocytes thus restrains the re-occlusion of the vessel. Similar to known restenosis inhibitors as e.g. rapamycin, the compounds of the invention inhibit cellular migration and interfere with extracellular matrix reorganization. In contrast to rapamycin, however, the compounds of the invention do not directly interfere with cell cycle signaling pathways. In consequence, they exhibit only minor effects on other cell functions and on cells surrounding the vessel injury, which do not actively migrate. This is of particular interest, as physiological reendothelialization, which is essential to successful wound healing of the vessel after surgery, should not be disturbed. Most common drugs used for preventing restenosis, however, inhibit cell proliferation and therefore impair reendothelialization (Inoue and Node 2009). Thus, specifically targeting assembly and disassembly of focal adhesions primarily effects cell migration and has only minor effects on other cell functions including proliferation. Therefore, the compounds according to the invention potentially inhibit restenosis while less affecting reendothelialization.

In a preferred embodiment, the compound is applied using a drug-eluting stent. This allows a local application of the compound of the invention at the side of injury. This reduces side effects, which are commonly observed to accompany systemic drug application. Additionally, the local application for allows achieving high concentrations of the compound at a particular site of interest without the need of high dose systemic applications.

In a further preferred embodiment, the drug eluting stent releases about 2 - 5 mg of the compound per day, to achieve effective local concentrations at the vessel wall.

In a further aspect, the invention relates to a compound of Formula I 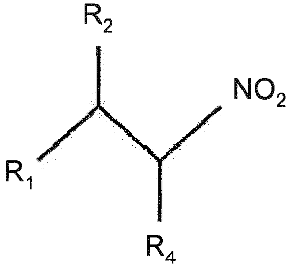 for use in treating cancer, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

Enhanced FAK expression and/or activity is found in a broad range of human cancers and is associated with resistance to chemotherapy. Moreover, FAK was found to play a central role in all steps of metastasis including proliferation, migration, survival and invasion. Thus, it has an important function in tumor progression and metastasis. Interestingly, it has been found that the endogenous FAK inhibitor FRNK efficiently blocks cell invasion and metastasis formation, at least in mice (Hauck et al. 2002b). However, common inhibitors of the FAK kinase activity were not able to inhibit all FAK functions, including cell invasion. The compounds of the invention, in contrast, specifically target the function of FAK in assembling focal adhesions and thereby also target the focal adhesion based signaling complexes regulating migration and invasion. Since the compounds of the invention specifically interfere with the assembly and disassembly of focal adhesions and thereby directly inhibit a cell's ability to migrate, they are particularly suitable for restricting and preventing metastasis. Therefore, in a preferred embodiment, the compound of the invention is used to prevent and/or treat metastasis.

In a preferred embodiment, targeting cancer comprises preventing and/or treating tumor growth and/or vascularization. With respect to tumor growth, it has been shown that inhibiting the kinase function of FAK exerts no effect on tumor growth. In contrast, the endogenous FAK inhibitor FRNK is able to block proliferation as well as to induce apoptosis. Similar to FRNK, the compounds of the invention specifically induce the delocalization of FAK from focal adhesions and thus do not only inhibit cell migration but are also expected to counteract tumor growth.

Regarding tumor vascularization it has been observed that tumor tissue, in particular when reaching a certain size, highly relies on vascularization to provide the tumor cells with sufficient nutrients and oxygen. To achieve this, tumor tissue often induces angiogenesis from pre-existing vasculature to achieve their own blood supply (Papetti and Hermann 2002). The formation of blood vessels not only involves cell proliferation and growth, but also cell adhesion and migration, wherein focal adhesions play a central role. By specifically inhibiting the assembly and disassembly of focal adhesions and thereby preventing cell migration, the vascularization of tumors can be directly targeted. Inhibiting vascularization of tumor tissue is known to efficiently reduce tumor size sometimes even inducing the death of the entire tumor.

In a preferred embodiment, the cancer is selected from the group consisting of breast cancer, prostate cancer, colon cancer, esopharyngeal cancer and ovarian carcinoma. FAK has recently been found to play a distinctive role in these types of cancers such that the compounds of the invention, which specifically target FAK localization in focal adhesions, are particularly suitable to target these kinds of cancers.

In a further aspect, the invention relates to a compound of Formula I 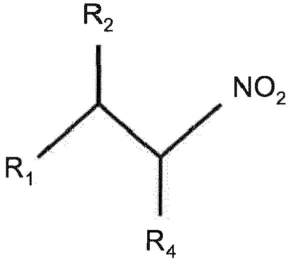 for use in preventing and/or treating cardiovascular diseases, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

Besides its role in angiogenesis, FAK was lately found to be involved in cardiovascular disorders as e.g. in regulating endothelial barrier function (Vadali et al. 2007). Likewise, FAK was implicated in strain-induced hypertrophy. Since both disorders were linked to the regulation of cell adhesion versus cell motility, the compounds of the invention are particularly suitable for treatment of these disorders, because they specifically target the assembly and disassembly of focal adhesions. Thereby they directly influence cell adhesion and cell motility.

In a preferred embodiment, a compound of Formula I 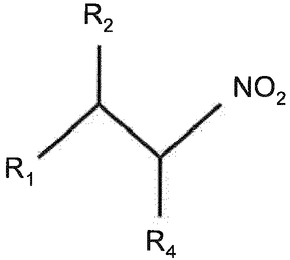 for use in treating inflammation, wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

The formation and destruction of focal adhesions play a major role in the immune system in general and during immune response, in particular. For example, individual steps of lymphocyte maturation occur at different places within the body, such that developing lymphocytes migrate through the body and settle by adhesion into specific niches for further maturation. Likewise, during the acute inflammatory response, cells move towards the inflammatory site where they adhere and settle down. In this process, the formation of adhesive points as well as the dynamic rearrangement of integrins and the actin cytoskeleton are essential. Both, FAK and paxillin, were found to play vital roles in controlling lymphocyte adhesion and migration. Moreover, the integrin signaling pathways involved in these processes are controlled and integrated at the points of focal adhesion (Romanova and Mushinski 2011). Given the specific effect of the compounds of the invention on FAK and paxillin localization at focal adhesions, the compounds are particularly suitable for interfering with processes of inflammation.

In a further aspect, the invention relates to a use of a compound of Formula I 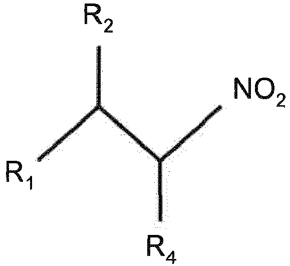 for inhibiting cellular migration and/or the turnover of focal adhesions *in vitro,* wherein R₁ is an aromatic group, R₂ is C-R₃, S-R₃, O-R₃, N-R₃, NH₂, or missing, R₃ is hydrogen, hydroxyl, amine, carboxyl, alkyl, cycloalkyl, heterocycloalkyl, aryl, heteroaryl, alkoxy, or cycloalkoxy, and R₄ is hydrogen or missing.

Observing living cells *in vitro* is often hampered by the motility and velocity of the cells, in particular of specific cell types as e.g. cells of the blood system. Applying the compounds of the invention, cell migration activity can be reduced, improving the optical investigation of the cells, in particular with respect to cell functions which are independent of cell adhesion and/or migration. Moreover, the compounds of the invention permit distinguishing between adhesion related mechanisms and other cell functions since they specifically target FAK and paxillin localization but do not interfere with e.g. the kinase function of FAK. Thus, the compounds of the invention provide valuable research tools, in particular for observations on the single cell level.

### Examples

### Identification of small molecules interfering with FAK localization at focal adhesions

Ectopic expression of FRNK as a dominant negative inhibitor of FAK effectively blocks FAK dependent events. This type of interference seems to be used physiologically for regulation of FAK function in certain cell types (Taylor et al. 2001). However, there is currently no possibility to instantaneously interfere with FAK localization. Therefore, the inventors sought to identify small molecules to disrupt FAK mediated protein-protein-interactions. Towards this line they set-up a High Content Screen based on FAK-/- MEFs re-expressing GFP-FAK to visualize FAK localization at focal adhesions. Cells were seeded on fibronectin and incubated to allow adherence and spreading. Prior to fixation, cell nuclei were Hoechst stained. FAK containing focal adhesions and cells per field of view were quantified using a custom-made macro for ImageJ. The macro identifies focal adhesions due to local maxima of fluorescence intensity in the GFP-channel. Cells were counted in the Hoechst channel based on a binary image. Closely connected cell nuclei were separated by a watershed algorithm. In order to evaluate the reproducibility of software-based quantification of focal adhesions per cell, 40 control wells of a multi-well plate containing semi-confluent layers of GFP-FAK MEFs were analyzed and a comparable number of focal adhesions per cell were identified in these cells **(****Figure 1****).** Next, as a proof of principle GFP-FAK MEFs were transduced to express mKate2-tagged FRNK. mKate2-FRNK localizes to focal adhesions, where it efficiently displaces GFP-FAK **(****Figure 2****).** In contrast, vinculin localization was not influenced by FRNK expression demonstrating that FAK dislocation was not due to a complete dissolution of focal adhesions. Indeed, software-based quantification of FAK-containing focal adhesions showed dramatically decreased numbers in case of FRNK expression, whereas the number of vinculin-containing focal adhesions per cell was comparable in both samples **(****Figure 3****).** Consequently, the software can correctly identify FAK displacement from focal adhesions.

In order to find small molecules which induce the specific delocalization of FAK, two commercial compound libraries with more than 17.000 entries were screened. GFP-FAK MEFs were seeded on fibronectin in multi-well plates and compounds were transferred to the cells with a robot to a final concentration of 40 µM. Cells were incubated for 4 h and nuclei were Hoechst stained prior to fixation. Cells were imaged with an automated microscope and data were analyzed with the ImageJ macro. In the first screening round the macro identified 108 hits, which were curated by visual inspection to exclude instances of dramatic cell rounding and/or detachment. In total, 38 compounds (primary hits) showed significant FAK displacement, while cells were still normally adherent and spread on the substrate. To validate these primary hits the inventors transduced the GFP-FAK MEFs to stably express mKate-vinculin, which serves as a FAK-independent marker of focal adhesions and facilitates indication of specific FAK displacement. On a first approach two compounds were identified, which induced FAK displacement within less than 1 h, while vinculin localization was not disturbed **(****Figure 4****).** The two compounds were trans-3-4-Bromo-2-(2-nitrovinyl)thiophene, hence forth Mb31 and 4-{[1-(4-Chlorophenyl)-2-nitroethyl]thio}aniline, hence forth Mb36. Strikingly, they share distinct structural characteristics since both compounds have a NO₂-group and an isosteric halogen-substituted phenyl- or thiophene-group (Figure 5).

### Mb31 and Mb36 displace FAK and paxillin efficiently within minutes

In order to investigate the kinetics of FAK displacement at focal adhesions a time series was performed. GFP-FAK MEFs were treated with 20 µM of Mb31 or Mb36 and fixed after different time points. Mb31 efficiently displaced FAK within 5 min, whereas in untreated cells FAK strongly localized to focal adhesions. Mb36 kinetic was slightly slower with FAK delocalization after 20 min. In addition, GFP-FAK MEFs were treated with increasing concentrations of Mb31 or Mb36 to determine the minimum concentration required to interfere with FAK localization. Untreated or solvent (DMSO) treated cells show the expected localization of FAK at focal adhesions. However, in Mb31 treated cells FAK localization in focal adhesions is strongly decreased. Already 2 µM of Mb31 were sufficient to induce FAK displacement. Similarly, in Mb36 treated cells no FAK could be detected in focal adhesions at 5 µM, although at 2 µM no effect was observed. In a second approach a narrower concentration range (1-5 µM) of Mb31 was applied revealing that about 4 µM were sufficient for efficient FAK displacement. Using ImageJ, the IC₅₀ values of Mb31 and Mb36, were found to be -2.0 µM for Mb31 and -4.1 µM for Mb36 (Figure 6). Due to the faster kinetic and lower IC₅₀ compared to Mb36 further studies were mainly performed using Mb31. Additional microscopic analyses with MEFs transiently expressing fluorescently tagged focal adhesion marker proteins confirmed rapid FAK displacement at 10 µM Mb31, but also demonstrated that FAK-binding partners such as paxillin, Grb2, and Crkll are also displaced together with FAK (Table 1). In contrast, vinculin localization and the focal adhesion localization of talin, α-actinin, and zyxin is not disturbed **(****Figure 5** and **Table 1).**

**Table 1 shows a summary of different focal adhesion proteins which lost or retained their focal adhesion association in the presence of Mb31.**

| **Displaced from focal adhesions** | **No effect on localization** |
|---|---|
| Focal adhesion kinase (FAK) | Talin |
| Paxillin | α-actinin |
| Grb2 | Vinculin |
| CRKII | Zyxin |

Mouse embryonic fibroblasts expressing the indicated proteins as fluorescent fusion proteins were incubated with 10 µM Mb31 or solvent alone for 30 min. Afterwards, the cells were fixed and the localization of the focal adhesion proteins was analyzed microscopically.

### Mb31 induced displacement of FAK is reversible and does not target FAK kinase activity

In order to analyze whether FAK or its binding partner paxillin are degraded after displacement, serum-starved GFP-FAK MEFs were stimulated with normal growth medium in presence of increasing concentrations of Mb31. After 30 min cells were lysed and protein levels were determined by immunoblotting. Neither for FAK nor for paxillin was any degradation observed **(****Figure 7****).** Furthermore, Y397 phosphorylation of FAK which serves as an indicator of FAK kinase activity was not influenced. This suggests that Mb31 does not interfere with the kinase activity of FAK. In line with this, *in vitro* activity of purified FAK kinase domain was not altered in presence of Mb31 **(****Figure 8****).** However, the FAK inhibitor PF431396 (Pfizer, New York, USA) completely inhibited FAK substrate phosphorylation. Considering, that paxillin and FAK are not degraded in Mb31 treated GFP-FAK MEFs the inventors further investigated whether FAK displacement is reversible. GFP-FAK MEFs were seeded on fibronectin and treated with 4 µM Mb31 for 1 h. Following compound washout, cells were incubated for 1 h and fixed. In line with previous results, FAK was completely displaced in Mb31 treated cells. However, after compound washout FAK relocalized to focal adhesions. To strengthen the hypothesis of FAK relocalization, in a second approach *de novo* protein synthesis was blocked with cycloheximide. Again, FAK containing focal adhesions could be clearly identified in the cell periphery. These results suggest that Mb31 does not target the kinase activity of FAK and that Mb31 induced effects are reversible.

### Mb31 does not impair cell viability

Toxicity assays were performed to investigate the effect of Mb31 on cell viability. This confirmed that Mb31 exhibits minor toxicity in concentrations, which efficiently reduce cell migration and block focal adhesion dynamics **(****Figure 9****).**

### Mb31 might displace FAK indirectly by targeting paxillin

The C-terminal focal adhesion targeting domain of FAK binds to paxillin and talin and is responsible for an efficient recruitment of FAK to focal adhesions. Therefore, it was further investigated whether Mb31 targets FAK directly or possibly in an indirect way by displacement of FAK binding proteins paxillin or talin, respectively. GFP-FAK MEFs were treated with 10 µM of Mb31 and analyzed for paxillin and talin localization via antibody staining. This revealed that talin localization is not influenced by Mb31 treatment **(Table 1).** However, similar to FAK paxillin was displaced from focal adhesions after 30 min, suggesting that Mb31 either targets paxillin directly or that FAK localization is important to stabilize paxillin localization. To analyze whether FAK and paxillin displacement occur with the same temporal pattern GFP-FAK MEFs were transiently transfected to express mKate-tagged paxillin and living cells were imaged after application of 10 µM of Mb31. Along with FAK, paxillin delocalized rapidly and within 10 min almost no paxillin and FAK remained at focal adhesions. Strikingly, in cells over-expressing paxillin higher Mb31 concentrations were necessary in order to achieve efficient FAK displacement. In non-transfected cells already 4 to 5 µM Mb31 were sufficient, whereas in cells with strong over-expression of paxillin even at 10 µM FAK and paxillin displacement was only partial. This indicates that the effect of Mb31 on FAK localization is inversely correlated with the amount of paxillin present within a cell. Consistent with the idea that paxillin localization at focal adhesions is directly influenced by Mb31, paxillin is efficiently displaced by Mb31 in FAK knockout MEFs **(****Figure 10****).** Still, vinculin localization was not altered in FAK-deficient MEFs treated with Mb31. These results suggest that Mb31 might induce FAK and paxillin displacement from focal adhesions by targeting the FAK-binding partner paxillin.

### Mb31 inhibits integrin-mediated cell migration

To further determine whether Mb31 interferes with cell motility, GFP-FAK MEFs were serum-stimulated and migration was quantified on a single cell basis **(Figures 11 and 12).** Upon serum starvation, cells were largely immobile (0.08 µm/min) **(****Figure 11A****).** Following addition of serum, cells started immediately to show random migratory behavior with a velocity of 0.29 µm/min **(****Figure 10B****).** In contrast, addition of serum together with 3 µM of Mb31 again resulted in immobile cells **(****Figure 11C****).** Similar experiments in the presence or absence of different concentrations of Mb31 revealed that this compound impaired cell motility already at a concentration of 2 µM (0.11 µm/min). At a Mb31 concentration of 3 µM (0.07 µm/min) cell motility was reduced to the level of unstimulated cells **(****Figure 11 D)****.** In addition, it was examined whether Mb31 allows interfering directly with the motility of migrating cells **(****Figures 12****).** GFP-FAK MEFs were serum-stimulated and migration of single cells was tracked for 5 h **(****Figure 12A****).** Mb31 was added and migration of the same cells was followed for additional 5 h. Clearly, cell motility was almost instantaneously blocked by concentrations of 3 µM or more of Mb31 (0.26 µm/min before treatment to 0.11 µm/min after treatment) **(****Figure 12B****).** Addition of solvent (DMSO) did not influence cell motility. **Figure 12C** shows the migration velocity of single cells with or without serum and the immediate reduction of migration speed upon addition of 3 or 4 µM Mb31. The same experiment was repeated and cells were either treated with the indicated amounts of Mb31 or a pharmacological kinase inhibitor of FAK (PF431396). Furthermore, cells were treated with *trans*-3-(4-bromothiophen-2-yl)acrylic acid (T34BA), a small control compound without FAK displacing ability. The results indicate that Mb31 is at least as effective as FAK kinase inhibitors in disrupting cell motility of fibroblasts **(Figure 13).** Due to their ability to block cell-migration, Mb31 was renamed Foot-Locker1 (FLocker1; for its ability to *"lock the feet of the cell*") and Mb36 was renamed FootLocker2 (FLocker2).

### Mb31 inhibits focal adhesion initiation and turnover

To investigate the dynamics of focal adhesions , GFP-FAK MEFs transiently expressing mKate-vinculin were serum-stimulated and imaged by TIRF microscopy. mKate-vinculin revealed the formation of protrusions at the leading edge of the cell, where multiple nascent adhesions are formed within an observation period of 40 minutes **(****Figure 14****;** arrows). However, after applying 5 µM Mb31 nascent adhesion formation was absent and mature adhesions were immobile as indicated by the static nature of vinculin-positive focal adhesions and the lack of newly formed nascent adhesions during 40 minutes **(****Figure 14****;** arrow). GFP-fluorescence from cytoplasmic localized FAK indicated that the cells still form protrusions, but fail to establish FAK- and vinculin-positive adhesions upon addition of Mb31. Furthermore, mouse embryonic fibroblasts were transfected with talinmCherry and cells were treated or not with 10 µM Mb31. To visualize protein dynamics at focal adhesions, mCherry fluorescence at ten different focal adhesion sites was recorded before and after local bleaching of talin-mCherry by laser irradiation to measure the fluorescence recovery after photobleaching (FRAP). In untreated cells, fluorescence was recovered by 40 s after the bleaching laser puls, indicating that talin molecules bleached at focal adhesion sites are replaced by unbleached talin molecules derived from the cytoplasmic pool **(****Figure 15A****).** This finding is in line with the reported constant turnover of focal adhesion constituents with a normal half life below 1 minute. In contrast, talin-associated fluorescence did not recover at bleached focal adhesions in cells treated with 10 µM Mb31, demonstrating that in the presence of Mb31 talin-mCherry at focal adhesion sites is not dynamically exchanged with the cytoplasmic pool of the protein suggesting that Mb31 severely interferes with focal adhesion turnover **(****Figure 15B****).** Thus, Mb31 interferes with both assembly and disassembly of focal adhesions and Mb31 might also be used as a tool to investigate protein dynamics at focal adhesions.

### FAK displacement by structurally related compounds

To determine whether compounds structurally related to Mb31 and Mb36 are likewise suitable to interfere with FAK localization at focal adhesions, mouse fibroblasts stably expressing GFP-FAK were treated for 30 min with 1, 10 or 20 µM of the following compounds: *trans*-3-hydroxy-β-nitrostyrene (THBN), *trans*-4-Chloro-β-nitrostyrene (T4CN), *trans*-β-nitrostyrene (TBN), 4-chlorocinnamic acid (4CA), and *trans*-3-(4-bromothiophen-2-yl)acrylic acid (T34BA) **(****Figures 16** and **17****).** The cells were analyzed by confocal microscopy for GFP-FAK localization. DMSO treatment served as a vehicle control. This analysis revealed that compounds having a basic structure comprising an aromatic system and a nitro group, e.g. 2-Nitrovinyl-thiophene or trans-beta-nitrostyrene mediate FAK displacement from focal adhesions. Additional substituents at the aromatic structures, such as halogen atoms or hydroxy groups may contribute to the reactivity of the compound, but are not essential. Based on these findings, the inventors have extended their structure activity-relationship studies and synthesized additional related compounds, which, similar to Mb31, displace FAK and paxillin at concentrations below 10µM (++), displace FAK and paxillin at concentrations above 10 µM (+), or lack FAK and paxillin displacing activity (-). A summary of the compounds investigated and their effect on FAK/paxillin localization is provided in Table 2. All these substances were then also nicknamed in the FLocker terminology, resulting in FLocker1 - FLocker24 (Table 2).

**Table 2: Overview of FootLocker nomenclature, their chemical notation/structure and the activity towards FAK/Paxillin displacement**

| **FootLocker** | **Compound** | **Activtiy** |
|---|---|---|
| 1 | 4-bromo-2-(2-nitrovinyl)thiophene (Mb31) | ++ |
| 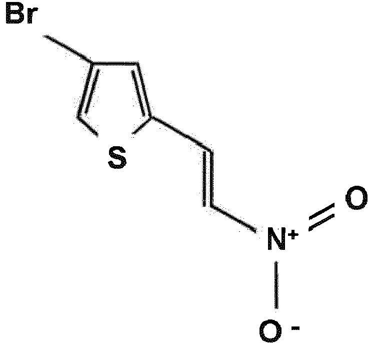 | | |
| 2 | 4-{[1-(4-chlorophenyl)-2-nitroethyl]thio}aniline (Mb36) | ++ |
| 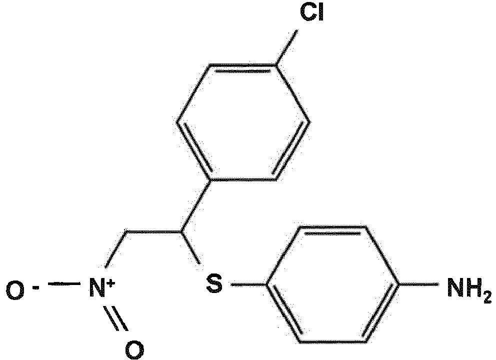 | | |
| 3 | (E)-3-(2-nitrovinyl)phenol | ++ |
| 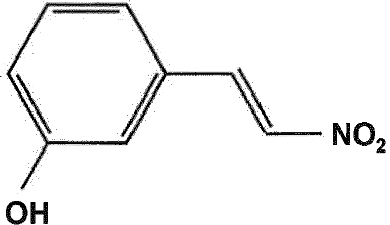 | | |
| 4 | (E)-1-methoxy-3-(2-nitrovinyl)benzene | ++ |
| 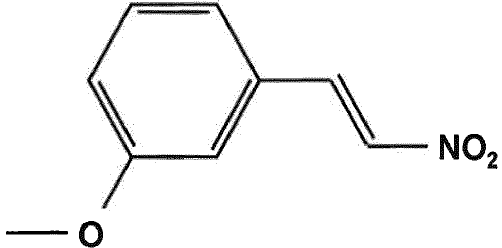 | | |
| 5 | (E)-ethyl 6-(4-(2-nitrovinyl)phenoxy)hexanoate | + |
| 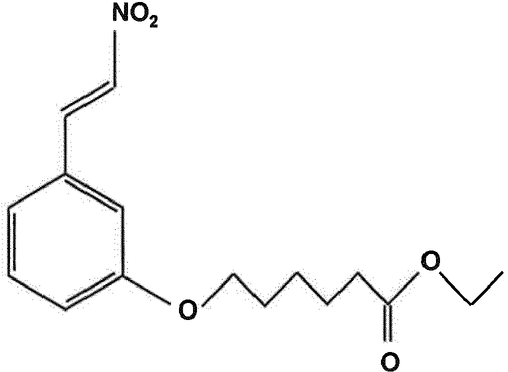 | | |
| 6 | N-(2-(2-(2-(2-azidoethoxy)ethoxy)ethoxy)ethyl)-7-(3-formylphenoxy)heptanamide | - |
| 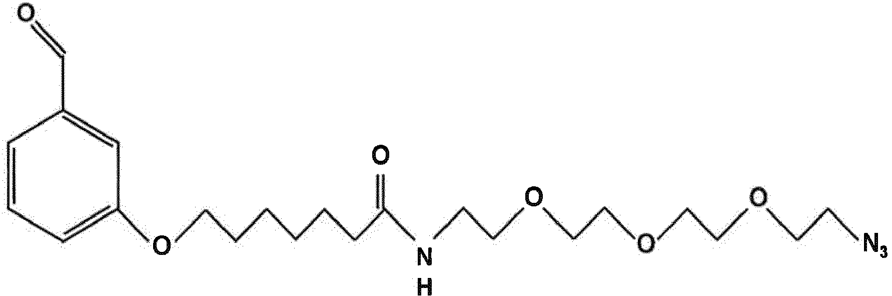 | | |
| 7 | (E)-N-(2-(2-(2-(2-azidoethoxy)ethoxy)ethoxy)ethyl)-7-(3-(2-nitrovinyl)phenoxy)heptanamide | + |
| 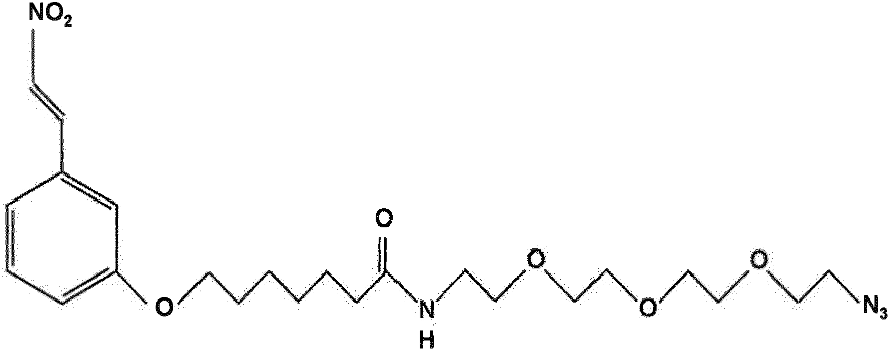 | | |
| 8 | (E)-2-(2-nitrovinyl)phenol | + |
| 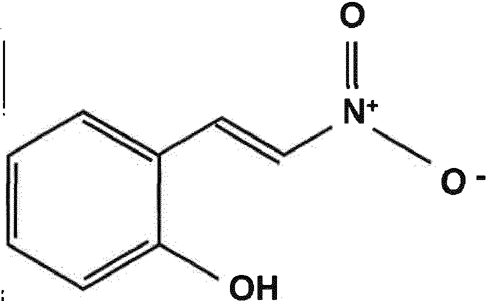 | | |
| 9 | (E)-1-fluoro-2-(2-nitrovinyl) benzene | ++ |
| 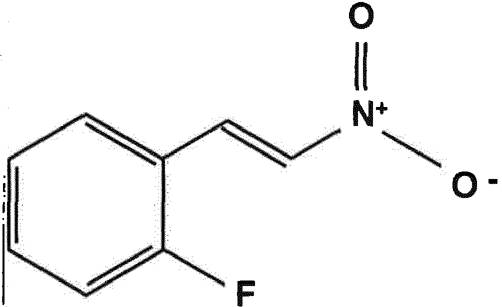 | | |
| 10 | (E)-1-methyl-4-(2-nitrovinyl) benzene | ++ |
| 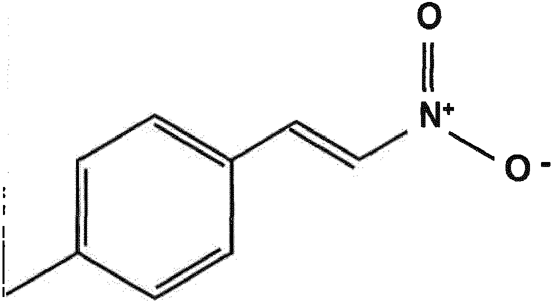 | | |
| 11 | (E)-1-bromo-3-(2-nitrovinyl)benzene | + |
| 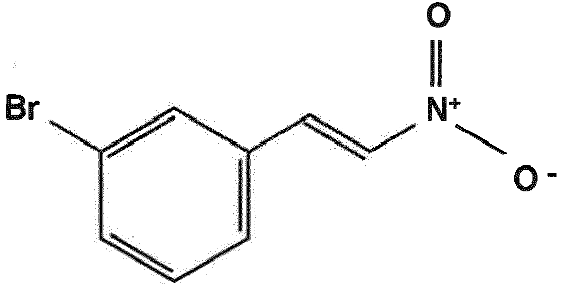 | | |
| 12 | (E)-1-chloro-3-(2-nitrovinyl)benzene | + |
| 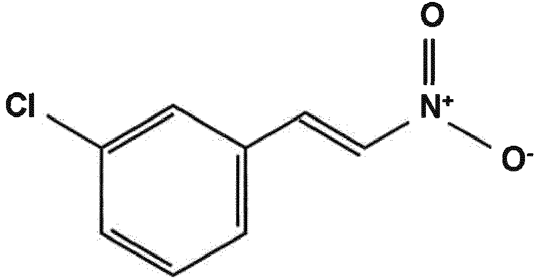 | | |
| 13 | (E)-1-nitro-3-(2-nitrovinyl)benzene | ++ |
| 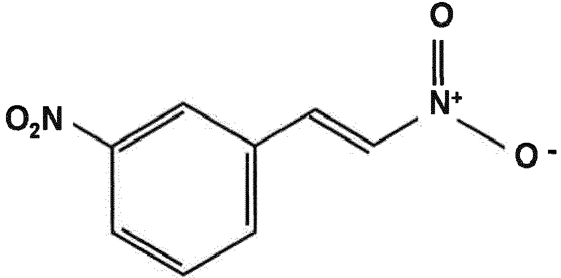 | | |
| 14 | (E)-1-fluoro-4-(2-nitrovinyl)benzene | ++ |
| 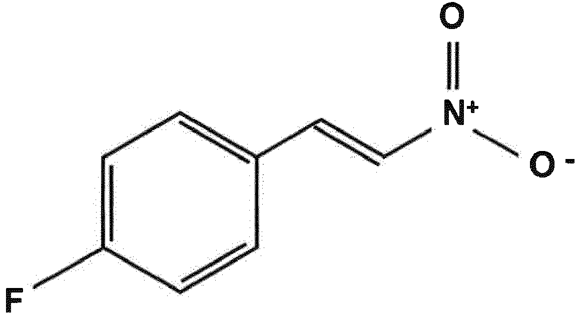 | | |
| 15 | (E)-(2-nitroprop-1-en 1-yl)benzene | + |
| 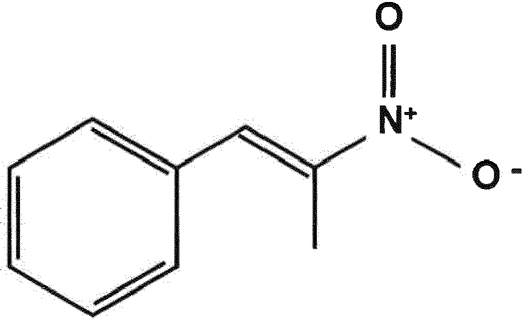 | | |
| 16 | 1-bromo-4-[(1E)-2-nitro-1-propenyl]benzene | ++ |
| 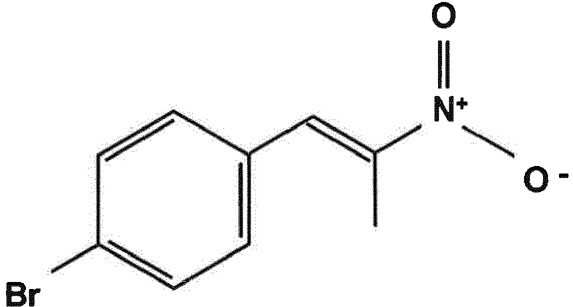 | | |
| 17 | 2-(2-Nitrovinyl)-furan | ++ |
| 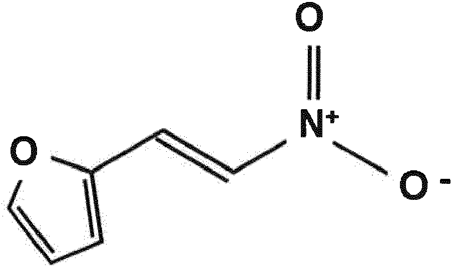 | | |
| 18 | 3-(2-nitro-vinyl)-pyridine | + |
| 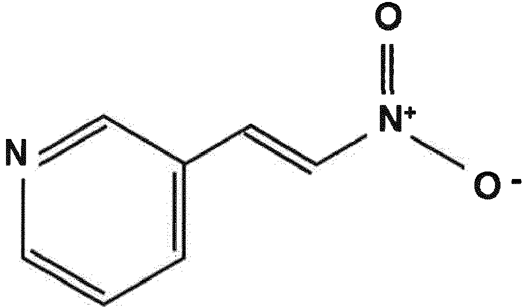 | | |
| 19 | 3-(2-nitrovinyl)-1-indole | - |
| 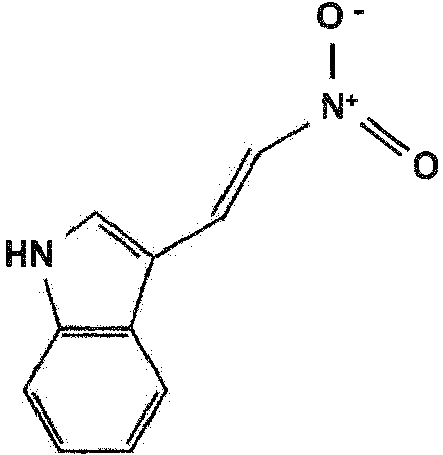 | | |
| 20 | 3-(2-nitrovinyl)-1-phenyl-1-indole | - |
| 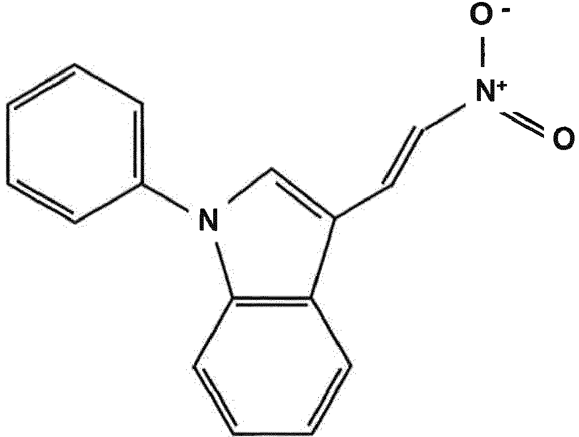 | | |
| 21 | styrene | - |
| 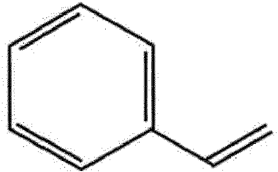 | | |
| 22 | 4-chlorocinnamaldehyde | - |
| 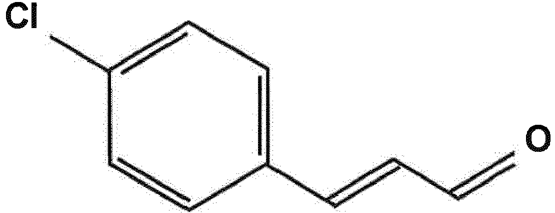 | | |
| 23 | Cinnamonitril | - |
| 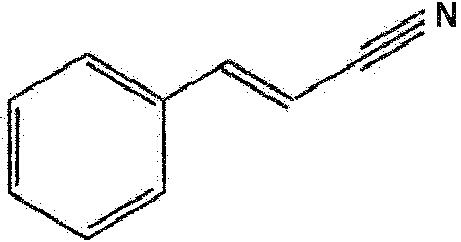 | | |
| 24 | *trans*-3-(4-Bromothiophen-2-yl)acrylic acid | - |
| 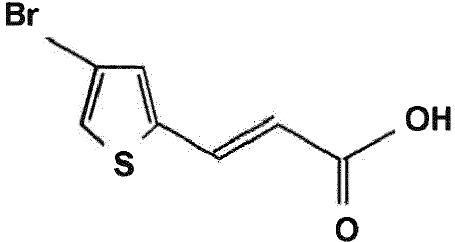 | | |

| | | |
|---|---|---|
| (++ indicates displacing effect at 5 µM and + at 20 µM; - indicates no observed effect) | | |

### Mb31 (FLocker 1) interferes with the migration of human breast cancer cells

To determine if the migration of transformed cells can be blocked by FLocker1 and related compounds, human breast carcinoma cells (MDA-MB-231) were used in *in vitro* wound healing assays. In addition to FLocker1, related compounds were employed with similar capabilities were named accordingly (FLocker 3, 9, 14) **(Figure 18A).** Cancer cells were first grown to confluency and then serum-starved. Upon introduction of a scratch-wound into the monolayer, cell migration was stimulated with the addition of serum-containing growth medium. Control cells remained in serum-free starvation medium **(****Figure 18B****).** Parallel to the addition of serum, cells received the indicated FLocker compounds at 3 µM or solvent (DMSO) and were observed for 25h. The ability of the cells to migrate and close the wound space was monitored by microscopy and quantified **(****Figure 18B** **and** **C****).** Clearly, application of FLocker compounds retarded the migration of the cancer cells, as visualized by the larger open space in the treated cultures. Taken together, these results demonstrate that Mb31 (FLocker1) induced displacement of FAK and paxillin strongly inhibits migration of normal and transformed cells.

### Recombinant DNA constructs

pLL3.7 mKate2 was designed by replacing EGFP cDNA in pLL3.7 EGFP (Addgene, Cambridge, MA) with mKate2 cDNA (Evrogen, Moskow, Russia) using Agel/EcoRI restriction sites. From mFAK cDNA the C-terminal region coding for aa693-1052 (FRNK) was amplified with the following primers: 5'-ATCGCTAGCACCATGGAATCCAGAAGACAGG-3' (sense) (SEQ ID NO.: 1) and 5'-ACTTGTACAGCTTCATGTGCATGTTCTCCTTAATCAGCTCGCTCACGCCG-CGTGTCTGCCCTAGC-3' (antisense) (SEQ ID NO.: 2). The resulting PCR fragment was cloned in pLL3.7 mKate by BsrGI/Nhel restriction sites.

Mouse vinculin WT cDNA (Illenberger, TU Braunschweig, Germany) was PCR-amplified with the following primers 5'-GAAGTTATCAGTCGACCCAGTGTTTCA-TACGC-3' (sense) (SEQ ID NO.: 3) and 5'- ATGGTCTAGAAAGCTTTTACTG-GTACCAGGGAG-3' (antisense) (SEQ ID NO.: 4). The resulting PCR fragment was cloned into pDNR-Dual using the In-Fusion PCR cloning Kit (Clontech, California, USA) and transferred to pmKate LoxP by Cre-mediated recombination.

Production of pmKate LoxP was described previously (Buntru et al. 2009). pBABE-Puro mKate-vinculin was designed by subcloning mKate-vinculin in pBABE-Puro (Addgene, Cambridge, MA) using BamHI/EcoRI restriction sites for pBABE Puro and Bcll/EcoRI restriction sites for pmKate vinculin. Human paxillin cDNA (Bershadsky, Weizmann Institute of Science, Rehovot, Israel) was amplified by PCR with the following primers 5'-ATCGTACTCGAGATATGGACGACCTCGACGC-3' (sense) (SEQ ID NO.: 5) and 5'-GTCAGCGAATTCCTAGCAGAAGAGCTTGAG-3' (antisense) (SEQ ID NO.: 6) and transferred to pBABE Puro mKate using Xhol/EcoRI restriction sites. Human FAK kinase domain was PCR-amplified from full-length cDNA (RZPD) with primers 5'-ATCGCGGCCGCACCATGCATCATCATCATCATCATGGCTCAACCAGG-GATTATGAG-3' (sense) (SEQ ID NO.: 7) and 5'-ACTGG-ATCCTTACCCATTCTTGAAATATTCAGGCTCTTCTTGCTGAGCCTTCTC-3' (antisense) (SEQ ID NO.: 8) and cloned in pVL1392 (BD Biosciences) using Notl/BamHI restriction sites.

### Cell culture, transfection of cells and transduction of cells

The human embryonic kidney cell line 293T (293T cells) and 293Φ Eco cells were grown in DMEM supplemented with 10% calf serum (CS) at 37°C, 5% CO₂. Mouse embryonic fibroblasts (MEFs) derived from FAK and p53 double-knockout mouse embryos reexpressing MEFs were cultured in DMEM with 10% fetal bovine serum (FBS), supplemented with non-essential amino acids and sodium pyruvate on gelatine-coated (0.1% gelatine in PBS) cell culture dishes. *Spodoptera frugiperda* (Sf9) cells were grown in IPL-41 medium (Genaxxon Bioscience, Ulm, Germany) supplemented with 0.37 g/l NaHCO₃, 2% yeastolate (Life Technologies GmbH, Darmstadt, Germany), 2% chemically defined lipid concentrate (Life Technologies GmbH, Darmstadt, Germany), penicillin/streptomycin and 10% FBS at 27°C. All cell lines were subcultured every 2-3 days. For transfection FAK-/- GFP-FAK cells were seeded at subconfluent density on fibronectin fragment (typelll-repeat 9-10, 1 µg/ml in PBS). Four hours later the cells were transfected with Lipofectamine 2000 (Invitrogen, Carslbad, CA) according to the manufacturer's instructions and used 24 h after transfection. 293T cells were transfected by calcium phosphate precipitation using 3 µg of appropriate cDNA and were used 48 h after transfection. For production of retroviral particles 293Φ Eco cells were seeded at subconfluent densities in two 10 cm culture dishes on poly-l-lysine (10 µg/ml in PBS) and transiently transfected with 15 µg pBABE Puro mKate-vinculin per dish using the calcium phosphate precipitation method. Medium was removed 8 h later and a glycerol shock was performed (15% glycerol in growth medium) for 3 min. Cells were washed once and incubated for 72 h at 37°C / 5% CO₂ in 6 ml growth medium per dish, supplemented with penicillin/streptomycin. For transduction fresh supernatant containing the viral particles was diluted with fibroblast growth medium and added to GFP-FAK MEFs seeded on fibronectin. 24 h later puromy-cin was added as a selection marker for transduced cells and based on the fluorescence a cell line was established from a single colony stably expressing mKate-vinculin.

### Immunofluorescence staining, fluorescence microscopy, live cell imaging

MEFs were seeded on fibronectin fragment (4 µg/ml in PBS) in multi-well plates (Greiner Bio-One) and incubated for 4 h to allow spreading. Cells were fixed and permeabilized with MeOH at -20°C after pre-fixation with 4% paraformaldehyde. Cells were blocked with 10% CS in PBS (blocking buffer) and incubated with primary antibodies (α-talin, α-paxillin or α-vinculin) in blocking buffer for 1 h at room temperature. Cells were washed three times with PBS and were incubated with appropriate fluorescently labeled secondary antibodies. Cells were washed again with PBS to remove excess antibodies and were imaged using a Leica AF6000LX fluorescence microscope. To analyze reversibility compound treated cells were washed three times with pre-warmed growth medium. After washout cells were incubated 1 h in presence of 10 µg/ml cycloheximide. Cells were washed with PBS, fixed with paraformaldehyde, washed again with PBS and directly imaged. For live cell imaging cells were seeded on fibronectin fragment (4 µg/ml in PBS) in cell culture dishes with glass-bottom insert. Cells were transfected 4 h later and were serum-starved for 16 h. Medium was replaced by normal growth medium and cells were imaged using a Leica AF6000LX equipped with a humidified incubation chamber at 37°C with 5% CO₂. For TIRF microscopy a Leica AF6000LX TIRF equipped with a 100x/1.46 NA Oil HCX PL Apo objective and an EMCCD camera (Cascadell:512) was used. All images were processed with ImageJ.

### MTT assay

MEFs were seeded on fibronectin (1 µg/ml in PBS) in starvation medium at a density of 1.4x10⁴ cells/well in triplicate and were serum-starved for 16 h. Medium was replaced with normal growth medium and after 5 h cells were treated with a dilution series of test compounds. After 5 h compound exposure cytotoxicity was evaluated using 0.5 mg/ml MTT (3-[4,5-Dimethyltiazol-2-yl] 2,5-diphenyltetrazolium bromide). Formazan was solubilized with DMSO and absorbance was measured at 550 nm (Varioskan Flash, Thermo Scientific).

### Expression and purification of recombinant proteins

Kinase domain of human FAK (aa 410-689, hFAK-KD) was expressed with an amino-terminal 6xHis-tag and a carboxy-terminal Nef epitope-tag. FAK residue 410 was changed from proline to glycine. Expression of the fusion protein was performed in Sf9 insect cells using the pVL1392 transfer vector and the BacuIoGold Transfection Kit (BD Biosciences, San Diego, USA). Cells were lysed in Sf9 lysis buffer (0.33% Tween20, 1 mM EDTA, 500 mM NaCl, 100 µM PMSF, 1 mM β-Mercaptoethanol in 25 mM TrisHCl, pH 7.5) for 20 min at 4°C. Subsequently, the lysate was cleared by centrifugation and hFAK-KD was purified from the soluble fraction under native conditions by FPLC using a HisTrapFFcrude 1 mL column (GE Healthcare, Munich, Germany)

### Cell migration assay

FAK-/- GFP-FAK cells were seeded on fibronectin (1 µg/ml in PBS) in starvation medium (0.5% BSA in DMEM) at low densities and were serum-starved for 16 h. To stimulate cell migration medium was replaced with normal growth medium with 10% FBS. Cells were treated with a dilution series of test compounds and were imaged for 8 h with a Leica AF6000LX fluorescence microscope (Leica, Mannheim, Germany) equipped with a humidified incubation chamber at 37°C with 5% CO₂. Single cell migration was quantified using the manual tracking plugin in ImageJ. In a similar setup cells were imaged 5 h before and additional 5 h after compound addition.

### ELISA-based in vitro kinase assay

A microtiter plate was coated with 2 µg/well of poly(Glu:Tyr) (4:1) copolymer (Sigma-Aldrich, Steinheim, Germany) as artificial FAK substrate and blocked with 2% BSA in PBS with 0.01% Tween20. Purified hFAK-KD together with 100 µM ATP were added in kinase buffer (125 mM NaCl, 48 mM MgCl₂, 50 mM HEPES, pH 7.5) in presence of various concentrations of test compounds and incubated for 1 h at 30°C. The reaction was stopped by the addition of EDTA and wells were extensively washed with 0.05% Tween20 in PBS. Phosphorylated substrate was detected by monoclonal anti-phosphotyrosine antibody followed by peroxidasecoupled goat anti-mouse antibody. TMB solution (0.5 mM 3,3',5,5'-Tetramethylbenzidine in 0.5% acetone, 4.5% ethanol and 1 mM H₂O₂ in 30 mM potassium citrate, pH 4.1) was added and the reaction was stopped with 2 M H₂SO₄ after a few minutes depending on the color development. The absorbance was measured at 450 nm.

### High content screening procedure

About 17.000 compounds (40 µM working concentration) were screened for their ability to displace GFP-FAK from focal adhesions (FA) using an automated microscope (ImageXpress Micro, Molecular Devices, Ismaning, Germany) equipped with a Nikon 20x/0.75 NA CFI Plan Apo objective. 6x10³ cells per well were seeded in 384 well-plates (Greiner Bio-One, Frickenhausen, Germany) on fibronectin fragment (4 µg/mL in PBS) and after adherence and complete spreading compounds were transferred to the cells by a pintool with a Tecan Freedom EVO workstation (Tecan, Männedorf, Switzerland). After 4 h compound exposure nuclei were Hoechst (Hoechst 33342, Invitrogen, Darmstadt, Germany) stained, cells were fixed with 4% paraformaldehyde and imaged. A ImageJ macro (Bioimaging center, University of Konstanz, Germany) was used to quantify focal adhesions per cell based on local fluorescence maxima in the GFP channel (size 5-70 pixel) and determination of cells per field in the Hoechst channel. In each well four fields were imaged. 38 initial hits were selected and analyzed in a second screening round using mKate-vinculin as a FAK-independent FA marker. Two structurally related compounds (Mb31 and Mb36) were identified which efficiently displace FAK from FAs while vinculin localization is not influenced. The IC₅₀ values were determined using the Hill slope model.

### References

EP 1 644 509
Buntru A, Zimmermann T, Hauck CR.; BMC Biol. 2009 Nov 25;7:81. doi: 10.1186/1741-7007-7-81. Fluorescence resonance energy transfer (FRET)-based subcellular visualization of pathogen-induced host receptor signaling.
Deramaudt TB, Dujardin D, Noulet F, Martin S, Vauchelles R, Takeda K, Rondé P. PLoS One. 2014 Mar 18; 9(3):e92059. doi: 10.1371/journal.pone.0092059. Altering FAK-paxillin interactions reduces adhesion, migration and invasion processes.
Golubovskaya VM, Nyberg C, Zheng M, Kweh F, Magis A, Ostrov D, Cance WG.; J Med Chem. 2008 Dec 11;51(23):7405-16. doi: 10.1021/jm800483v. A small molecule inhibitor, 1,2,4,5-benzenetetraamine tetrahydrochloride, targeting the y397 site of focal adhesion kinase decreases tumor growth.
Golubovskaya VM, Figel S, Ho BT, Johnson CP, Yemma M, Huang G, Zheng M, Nyberg C, Magis A, Ostrov DA, Gelman IH, Cance WG.; Carcinogenesis. 2012 May;33(5):1004-13. doi: 10.1093/carcin/bgs120. Epub 2012 Mar 7.; A small molecule focal adhesion kinase (FAK) inhibitor, targeting Y397 site: 1-(2-hydroxyethyl)-3, 5, 7-triaza-1-azoniatricyclo [3.3.1.1(3,7)]decane; bromide effectively inhibits FAK autophosphorylation activity and decreases cancer cell viability, clonogenicity and tumor growth in vivo.
Hagel M, George EL, Kim A, Tamimi R, Opitz SL, Turner CE, Imamoto A, Thomas SM.; Mol Cell Biol. 2002 Feb;22(3):901-15.; The adaptor protein paxillin is essential for normal development in the mouse and is a critical transducer of fibronectin signaling.
Hauck CR, Sieg DJ, Hsia DA, Loftus JC, Gaarde WA, Monia BP, Schlaepfer DD.; Cancer Res. 2001 Oct 1;61(19):7079-90.; Inhibition of focal adhesion kinase expression or activity disrupts epidermal growth factor-stimulated signaling promoting the migration of invasive human carcinoma cells.
Hauck CR, Hsia DA, Schlaepfer DD.; IUBMB Life. 2002a Feb;53(2):115-9. The focal adhesion kinase--a regulator of cell migration and invasion.
Hauck CR, Hsia DA, Puente XS, Cheresh DA, Schlaepfer DD.; EMBO J. 2002b Dec 2;21(23):6289-302. FRNK blocks v-Src-stimulated invasion and experimental metastases without effects on cell motility or growth.
Inoue T, Node K. ; Circ J. 2009 Apr;73(4):615-21. Epub 2009 Mar 13. Molecular basis of restenosis and novel issues of drug-eluting stents.
Kanchanawong P, Shtengel G, Pasapera AM, Ramko EB, Davidson MW, Hess HF, Waterman CM.; Nature. 2010 Nov 25;468(7323):580-4. doi: 10.1038/nature09621.; Nanoscale architecture of integrin-based cell adhesions.
Liu TJ, LaFortune T, Honda T, Ohmori O, Hatakeyama S, Meyer T, Jackson D, de Groot J, Yung WK.; Mol Cancer Ther. 2007 Apr;6(4):1357-67. ; Inhibition of both focal adhesion kinase and insulin-like growth factor-I receptor kinase suppresses glioma proliferation in vitro and in vivo.
Papetti M, Herman IM.; Am J Physiol Cell Physiol. 2002 May;282(5):C947-70. Mechanisms of normal and tumor-derived angiogenesis.
Romanova LY, Mushinski JF.; Cell Adh Migr. 2011 Nov-Dec;5(6):457-62. doi: 10.4161/cam.5.6.18219. Central role of paxillin phosphorylation in regulation of LFA-1 integrins activity and lymphocyte migration.
Schaller MD.; J Cell Sci. 2010 Apr 1;123(Pt 7):1007-13. doi: 10.1242/jcs.045112. Cellular functions of FAK kinases: insight into molecular mechanisms and novel functions.
Schiller HB1, Friedel CC, Boulegue C, Fässler R.; EMBO Rep. 2011 Mar;12(3):259-66. doi: 10.1038/embor.2011.5. Epub 2011 Feb 11.; Quantitative proteomics of the integrin adhesome show a myosin II-dependent recruitment of LIM domain proteins.
Sieg DJ, Hauck CR, Ilic D, Klingbeil CK, Schaefer E, Damsky CH, Schlaepfer DD. Nat Cell Biol. 2000 May;2(5):249-56. FAK integrates growth-factor and integrin signals to promote cell migration.
Taylor JM, Mack CP, Nolan K, Regan CP, Owens GK, Parsons JT.; Mol Cell Biol. 2001 Mar;21(5):1565-72. Selective expression of an endogenous inhibitor of FAK regulates proliferation and migration of vascular smooth muscle cells.
Vadali K, Cai X, Schaller MD.; IUBMB Life. 2007 Nov;59(11):709-16. Focal adhesion kinase: an essential kinase in the regulation of cardiovascular functions.
Wade R, Bohl J, Vande Pol S.; Oncogene. 2002 Jan 3;21(1):96-107.; Paxillin null embryonic stem cells are impaired in cell spreading and tyrosine phosphorylation of focal adhesion kinase.

## Claims

1. A compound of Formula I 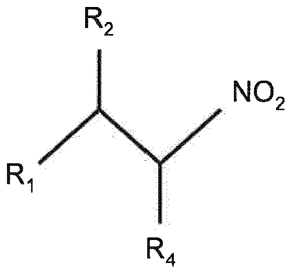 for use in inhibiting cell migration, wherein
R₁ is an aromatic group,
R₂ is C-R₃, S-R₃, O-R₃, N-R₃, NH₂, or missing,
R₃ is hydrogen, hydroxyl, amine, carboxyl, alkyl, cycloalkyl, heterocycloalkyl, aryl, heteroaryl, alkoxy, or cycloalkoxy, and
R₄ is hydrogen or missing.

2. The compound of claim 1, wherein R₁ and/or R₃ are independently an aryl or heteroaryl, preferably the aryl or heteroaryl is monocyclic, bicyclic or tricyclic.

3. The compound of claim 1 or 2, wherein the aryl or heteroaryl is substituted and the substituent is selected from the group consisting of hydroxyl, alkyl, preferably methyl, ethyl or butyl, aryl, preferably monocyclic, bicyclic or tricyclic aryl, heteroaryl, sulfanyl and halogen, preferably Br, Cl or F.

4. The compound of any of the preceding claims, wherein R₁ is a monocyclic, optionally substituted aryl or heteroaryl and R₂ and R₄ are missing.

5. The compound of any of the preceding claims, wherein the IC₅₀ of the compound is about 0,1 - about 0,5 µM, preferably about 0,1 - about 0,4 µM, more preferred about 0,1 - about 0,25 µM.

6. The compound of any of the preceding claims, wherein the compound is selected from the group consisting of 4-bromo-2-(2-nitrovinyl) thiophene (Mb31), 4-{[1-(4-chlorophenyl)-2-nitroethyl]thio}aniline (Mb36), (E)-3-(2-nitrovinyl)pieno, (E)-1-methoxy-3-(2-nitrovinyl)benzene, (E)-ethyl 6-(4-(2-nitrovinyl)phenoxy)hexanoate, (E)-N-(2-(2-(2-(2-azidoethoxy)ethoxy)-ethoxy)ethyl)-7-(3-(2-nitrovinyl)phenoxy)heptanamide, (E)-2-(2-nitrovinyl)pieno, (E)-1-fluoro-2-(2-nitrovinyl) benzene, (E)-1-methyl-4-(2-nitrovinyl) benzene, (E)-1-bromo-3-(2-nitrovinyl)benzene, (E)-1-chloro-3-(2-nitrovinyl)benzene, (E)-1-nitro-3-(2-nitrovinyl)benzene, (E)-1-fluoro-4-(2-nitrovinyl)benzene, (E)-(2-nitroprop-1-en 1-yl)benzene, 1-bromo-4-[(1E)-2-nitro-1-propenyl]benzene, 2-(2-Nitrovinyl)-furan, and 3-(2-nitro-vinyl)-pyridine.

7. The compound of any of the preceding claims, wherein the cell is a eukaryotic cell, preferably a mammalian cell, more preferred a human cell.

8. The compound of claim 7, wherein the cell is a vascular muscle cell, a cell of the immune system and/or a cancer cell.

9. A compound of Formula I 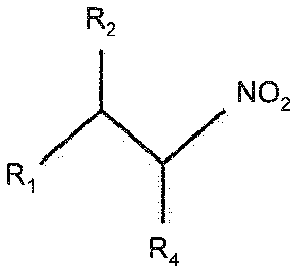 for use in preventing and/or treating restenosis, wherein
R₁ is an aromatic group,
R₂ is C-R₃, S-R₃, O-R₃, N-R₃, NH₂, or missing,
R₃ is hydrogen, hydroxyl, amine, carboxyl, alkyl, cycloalkyl, heterocycloalkyl, aryl, heteroaryl, alkoxy, or cycloalkoxy, and
R₄ is hydrogen or missing.

10. The compound of claim 9, wherein the compound is applied using a drug-eluting stent.

11. A compound of Formula I 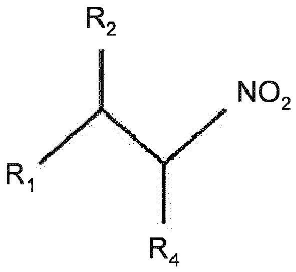 for use in treating cancer, wherein
R₁ is an aromatic group,
R₂ is C-R₃, S-R₃, O-R₃, N-R₃, NH₂, or missing,
R₃ is hydrogen, hydroxyl, amine, carboxyl, alkyl, cycloalkyl, heterocycloalkyl, aryl, heteroaryl, alkoxy, or cycloalkoxy, and
R₄ is hydrogen or missing.

12. The compound of claim 11 for use in preventing and/or treating metastasis.

13. The compound of claim 11 or 12 for use in preventing and/or treating tumor growth and/or vascularization.

14. A compound of Formula I 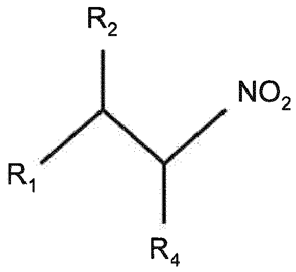 for use in preventing and/or treating cardiovascular diseases, wherein
R₁ is an aromatic group,
R₂ is C-R₃, S-R₃, O-R₃, N-R₃, NH₂, or missing,
R₃ is hydrogen, hydroxyl, amine, carboxyl, alkyl, cycloalkyl, heterocycloalkyl, aryl, heteroaryl, alkoxy, or cycloalkoxy, and
R₄ is hydrogen or missing.

15. Use of a compound of Formula I 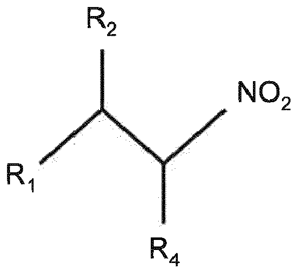 for inhibiting cellular migration and/or the turnover of focal adhesions *in vitro,* wherein
R₁ is an aromatic group,
R₂ is C-R₃, S-R₃, O-R₃, N-R₃, NH₂, or missing,
R₃ is hydrogen, hydroxyl, amine, carboxyl, alkyl, cycloalkyl, heterocycloalkyl, aryl, heteroaryl, alkoxy, or cycloalkoxy, and
R₄ is hydrogen or missing.


SEQUENCE LISTING

<110> Universität Konstanz

<120> Small molecule compounds displacing focal adhesion core components

<130> T30046

<160> 8

<170> BiSSAP 1.3

<210> 1
<211> 31
<212> DNA
<213> Homo sapiens

<400> 1
atcgctagca ccatggaatc cagaagacag g                                    31


<210> 2
<211> 65
<212> DNA
<213> Homo sapiens

<400> 2
acttgtacag cttcatgtgc atgttctcct taatcagctc gctcacgccg cgtgtctgcc     60

ctagc                                                                 65


<210> 3
<211> 32
<212> DNA
<213> Homo sapiens

<400> 3
gaagttatca gtcgacccag tgtttcatac gc                                   32


<210> 4
<211> 33
<212> DNA
<213> Homo sapiens

<400> 4
atggtctaga aagcttttac tggtaccagg gag                                  33


<210> 5
<211> 31
<212> DNA
<213> Homo sapiens

<400> 5
atcgtactcg agatatggac gacctcgacg c                                    31


<210> 6
<211> 30
<212> DNA
<213> Homo sapiens

<400> 6
gtcagcgaat tcctagcaga agagcttgag                                      30


<210> 7

<211> 56
<212> DNA
<213> Homo sapiens

<400> 7
atcgcggccg caccatgcat catcatcatc atcatggctc aaccagggat tatgag          56


<210> 8
<211> 54
<212> DNA
<213> Homo sapiens

<400> 8
actggatcct tacccattct tgaaatattc aggctcttct tgctgagcct tctc          54